# EUROPEAN PATENT APPLICATION

(11) **EP 1 944 024 A1**
(43) Date of publication of application: **16.07.2008**
(21) Application number: 06823024.2
(22) Date of filing: 06.11.2006
(51) Int. Cl.: A61K 31/343, A61K 31/4155, A61K 31/4178, A61K 31/422, A61K 31/427, A61K 31/439, A61K 31/454, A61K 31/496, A61P 3/10, A61P 9/00, A61P 9/10, A61P 13/12, A61P 27/02, A61P 39/06, A61P 43/00, C07D 405/06, C07D 405/12, C07D 405/14, C07D 413/12, C07D 417/12, C07D 471/08

(54) **O-SUBSTITUTED ANILINE DERIVATIVE AND ANTIOXIDANT DRUG**

(30) Priority: 04.11.2005 JP 2005321612
(71) Applicant: NIPPON SODA CO., LTD., Chiyoda-ku, Tokyo 100-8165 (JP)
(72) Inventor: TSUBOKURA, Shiro, Odawara-shi Kanagawa 250-0280 (JP); UMEDA, Nobuhiro, Odawara-shi Kanagawa 250-0280 (JP); UCHIDA, Seiichi, Odawara-shi Kanagawa 250-0280 (JP)
(74) Representative: Wibbelmann, Jobst
(86) International application number: PCT/JP2006/322111
(87) International publication number: WO 2007/052794

(57) **Abstract**

A compound or a salt thereof that exhibits effective antioxidant activity in the treatment of ischemic organ disorders such as arteriosclerosis, myocardial infarction and brain infarction, in the treatment of diseases caused by oxidative cell damage such as renal disease, and in the inhibition of retinal lesions caused by oxidation due to the effects of light or the like. Also provided is an antioxidant drug containing at least one of the compounds or salts as an active ingredient.

A compound represented by a formula (1) or a salt thereof, and an antioxidant drug containing at least one of the compound and the salt as an active ingredient. 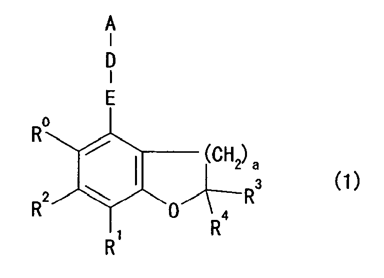 {In the formula (1):
a represents either 1 or 2,
R⁰ represents an unsubstituted or substituted amino group,
R¹ to R⁴ each represent, independently, a hydrogen atom or an alkyl group,
E represents an unsubstituted or substituted alkylene chain,
D represents a single bond, oxygen atom, unsubstituted or substituted nitrogen atom, sulfur atom, sulfinyl group, sulfonyl group, carbonyl group, carbonylamino group or aminocarbonyl group, and
A represents an unsubstituted or substituted aromatic hydrocarbon group, unsubstituted or substituted heterocyclic group, unsubstituted or substituted aralkyl group, or unsubstituted or substituted heteroaralkyl group.}

## Description

### TECHNICAL FIELD

The present invention relates to a novel ortho-substituted aniline derivative, and an antioxidant drug that uses this compound as an active ingredient.
Priority is claimed on Japanese Patent Application No. 2005-321612, filed November 4, 2005, the content of which is incorporated herein by reference.

### BACKGROUND ART

In recent years, it has become clear that *in vivo* lipid peroxidation and the associated radical reactions have a variety of adverse effects on the living organism due to membrane damage and cell damage and the like. As a result, various tests have been conducted on the use of antioxidant drugs and drugs that inhibit lipid peroxidation as potential medications. However, although many antioxidant drugs have been researched, they have suffered from problems such as weak activity or the existence of side effects, and they are not entirely satisfactory from a practical perspective.

Examples of compounds that exhibit an antioxidant activity and have a similar structure to the compound of the present invention include compounds represented by a formula shown below:

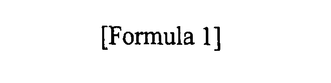

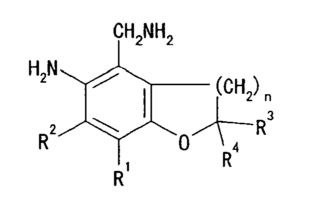

(wherein, R¹ to R⁴ each represent, independently, a hydrogen atom or a C₁ to C₆ alkyl group, and n represents an integer of either 1 or 2). A representative compound is the compound represented by the formula shown below (see patent reference 1).

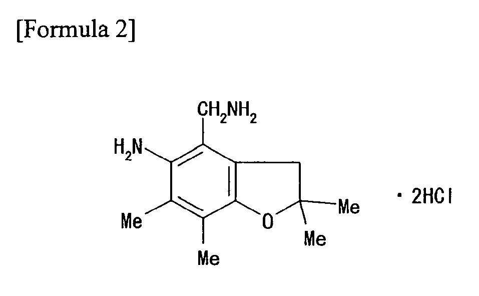

However, because the compound exhibits a low blood concentration following administration, and does not necessarily have satisfactory migration properties, it does not provide an adequate medicinal effect when used as an orally-administered drug.
[Patent Document 1]
International Patent Publication No. 2004/092153 pamphlet

### DISCLOSURE OF INVENTION

The inventors of the present invention surmised that the reason that the efficacy of existing antioxidant drugs was unsatisfactory upon oral administration was due to the drug either not reaching the target site, or losing its activity prior to reaching the target site. Accordingly, an object of the present invention is to provide an antioxidant drug that exhibits favorable organ migration properties, passes particularly readily through the blood-brain barrier or blood-retina barrier, and even when administered orally, produces a high post-administration blood concentration and satisfactory migration properties.

As a result of intensive investigation aimed at achieving the above object, the inventors of the present invention focused their attention on the aminomethyl group at position-4 of the representative compound disclosed in the patent reference 1, and discovered that when a functional group such as an aromatic hydrocarbon group or a heterocyclic group was introduced via some type of spacer, the resulting compound exhibited an excellent *in vivo* antioxidant action even following oral administration, and they were therefore able to compete the present invention.

Namely, a first aspect of the present invention provides a compound represented by a formula (1), or a salt thereof.

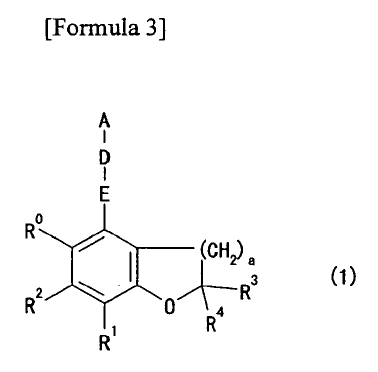

{In the formula (1), a represents either 1 or 2,
R⁰ represents an unsubstituted or substituted amino group,
R¹ to R⁴ each represent, independently, a hydrogen atom or an alkyl group,
E represents an unsubstituted or substituted alkylene chain,
D represents a single bond, oxygen atom, unsubstituted or substituted nitrogen atom, sulfur atom, sulfinyl group, sulfonyl group, carbonyl group, carbonylamino group or aminocarbonyl group, and
A represents an unsubstituted or substituted aromatic hydrocarbon group, unsubstituted or substituted heterocyclic group, unsubstituted or substituted aralkyl group, or unsubstituted or substituted heteroaralkyl group.}

A second aspect of the present invention provides an antioxidant drug that includes, as an active ingredient, at least one material selected from the group consisting of compounds represented by the formula (1) and salts thereof.

A compound of the present invention represented by the above formula (1) or a salt thereof exhibits effective antioxidant activity in the treatment of ischemic organ disorders such as arteriosclerosis, myocardial infarction and brain infarction, and in the treatment of diseases caused by oxidative cell damage such as renal disease. Furthermore, the compound or salt can also effectively inhibit retinal lesions caused by oxidation due to the effect of light or the like. Furthermore, the antioxidant drug of the present invention can also be used as an oxidative lesion inhibitor with minimal side effects, a lipoxygenase inhibitor, a 20-HETE synthase inhibitor, a drug for treating renal disease, cerebrovascular disease or cardiovascular disease, and a drug for treating brain infarction and the like.

### BEST MODE FOR CARRYING OUT THE INVENTION

A more detailed description of the present invention is provided below.

### 1) Compounds represented by Formula (1) and Salts thereof

In a compound represented by the above formula (1), R⁰ represents an unsubstituted or substituted amino group, and an unsubstituted amino group is preferred.

Specific examples of substituents within R⁰ include acyl groups such as a formyl group, acetyl group or benzoyl group; alkoxycarbonyl groups such as a methoxycarbonyl group, ethoxycarbonyl group, n-propoxycarbonyl group, isopropoxycarbonyl group, n-butoxycarbonyl group, s-butoxycarbonyl group, isobutoxycarbonyl group or t-butoxycarbonyl group; alkyl groups such as a methyl group, ethyl group, n-propyl group, isopropyl group, n-butyl group, s-butyl group, isobutyl group, t-butyl group, n-pentyl group or n-hexyl group; aromatic hydrocarbon groups such as a phenyl group, 1-naphthyl group, 2-naphthyl group, 1-anthryl group, 2-anthryl group or 9-anthryl group; and aralkyl groups such as a benzyl group, 1-phenylethyl group, 2-phenylethyl group, 1-methylnaphthyl group, 2-methylnaphthyl group, 1-ethylnaphthyl group or 2-ethylnaphthyl group. Functional groups that are able to dissociate or decompose by *in vivo* metabolism are preferred.

R¹ to R⁴ each represent a hydrogen atom or an alkyl group, and an alkyl group is preferred.

Specific examples of the alkyl groups of R¹ to R⁴ include the same groups as those listed above as specific examples of alkyl groups that can function as a substituent for the amino group of R⁰, and C₁ to C₆ alkyl groups are preferred.

E represents an unsubstituted or substituted alkylene chain, is preferably an unsubstituted alkylene chain, and is preferably represented by -(CH₂)ₙ- (wherein, n represents an integer of 1 to 6).

Specific examples of substituents on the alkylene chain include a hydroxyl group; a thiol group; halogen atoms such as a fluorine atom, chlorine atom, bromine atom or iodine atom; a cyano group; a nitro group; a formyl group; unsubstituted or substituted amino groups such as an amino group, methylamino group, benzylamino group, anilino group, dimethylamino group, diethylamino group or phenylethylamino group; alkyl groups such as a methyl group, ethyl group, n-propyl group, isopropyl group, n-butyl group, s-butyl group, isobutyl group, t-butyl group, n-pentyl group or n-hexyl group; alkenyl groups such as a vinyl group or allyl group; alkynyl groups such as an ethynyl group, 1-propynyl group or propargyl group; alkoxy groups such as a methoxy group, ethoxy group, n-propoxy group, isopropoxy group, n-butoxy group, s-butoxy group, isobutoxy group or t-butoxy group; alkenyloxy groups such as a vinyloxy group or allyloxy group; alkynyloxy groups such as an ethynyloxy group or propargyloxy group; aryloxy groups such as a phenoxy group; haloalkyl groups such as a chloromethyl group, fluoromethyl group, bromomethyl group, dichloromethyl group, difluoromethyl group, dibromomethyl group, trichloromethyl group, trifluoromethyl group, bromodifluoromethyl group, 1,1,1-trifluoroethyl group, 1-chloroethyl group, 2-chloroethyl group, 1-bromoethyl group or pentafluoroethyl group; haloalkoxy groups such as a fluoromethoxy group, chloromethoxy group, bromomethoxy group, difluoromethoxy group, dichloromethoxy group, dibromomethoxy group, trifluoromethoxy group, trichloromethoxy group, tribromomethoxy group, trifluoroethoxy group, pentafluoroethoxy group or pentafluoro-n-propoxy group; alkylthiocarbonyl groups such as a methylthiocarbonyl group, ethylthiocarbonyl group, n-propylthiocarbonyl group, isopropylthiocarbonyl group, n-butylthiocarbonyl group, isobutylthiocarbonyl group, s-butylthiocarbonyl group or t-butylthiocarbonyl group; alkylcarbonylamino groups such as a methylcarbonylamino group, ethylcarbonylamino group, n-propylcarbonylamino group or isopropylcarbonylamino group; alkoxycarbonylamino groups such as a methoxycarbonylamino group, ethoxycarbonylamino group, n-propoxycarbonylamino group or isopropoxycarbonylamino group; alkoxycarbonyl groups such as a methoxycarbonyl group, ethoxycarbonyl group, n-propoxycarbonyl group, isopropoxycarbonyl group, n-butoxycarbonyl group or t-butoxycarbonyl group; aromatic hydrocarbon groups such as a phenyl group, 1-naphthyl group or 2-naphthyl group; unsaturated 5-membered heterocyclic groups such as a furan-2-yl group, furan-3-yl group, thiophen-2-yl group, thiophen-3-yl group, pyrrol-2-yl group, pyrrol-3-yl group, oxazol-2-yl group, oxazol-4-yl group, oxazol-5-yl group, thiazol-2-yl group, thiazol-4-yl group, thiazol-5-yl group, isooxazol-3-yl group, isooxazol-4-yl group, isooxazol-5-yl group, isothiazol-3-yl group, isothiazol-4-yl group, isothiazol-5-yl group, imidazol-2-yl group, imidazol-4-yl group, imidazol-5-yl group, pyrazol-3-yl group, pyrazol-4-yl group, pyrazol-5-yl group, 1,3,4-oxadiazol-2-yl group, 1,3,4-thiadiazol-2-yl group, 1,2,3-triazol-4-yl group, 1,2,4-triazol-3-yl group or 1,2,4-triazol-5-yl group; unsaturated 6-membered heterocyclic groups such as a pyridin-2-yl group, pyridin-3-yl group, pyridin-4-yl group, pyridazin-3-yl group, pyridazin-4-yl group, pyrazin-2-yl group, pyrimidin-2-yl group, pyrimidin-4-yl group, pyrimidin-5-yl group, 1,3,5-triazin-2-yl group or 1,2,4-triazin-3-yl group; saturated heterocyclic groups such as a tetrahydrofuran-2-yl group, tetrahydrofuran-4-yl group, piperidin-3-yl group, pyrrolidin-2-yl group, morpholino group or piperidino group; alkylthio groups such as a methylthio group, ethylthio group or t-butylthio group; alkylsulfonyl groups such as a methylsulfonyl group, ethylsulfonyl group or t-butylsulfonyl group; alkenylsulfonyl groups such as an allylsulfonyl group; alkynylsulfonyl groups such as a propargylsulfonyl group; and arylsulfonyl groups such as a phenylsulfonyl group.

D represents a single bond, oxygen atom, unsubstituted or substituted nitrogen atom, sulfur atom, sulfinyl group, sulfonyl group, carbonyl group, carbonylamino group or aminocarbonyl group.

Specific examples of substituents on the nitrogen atom of D include the same groups as those listed within a portion of the specific substituent list provided for the alkylene chain of E.

A represents an unsubstituted or substituted aromatic hydrocarbon group, unsubstituted or substituted heterocyclic group, unsubstituted or substituted aralkyl group, or unsubstituted or substituted heteroaralkyl group.

Specific examples of the aromatic hydrocarbon of A include the same groups as those listed within a portion of the specific substituent list provided for the alkylene chain of E.

There are no particular restrictions on the heterocyclic group of A, provided it includes at least one hetero atom such as an oxygen atom, nitrogen atom or sulfur atom within the ring structure, and specific examples include unsaturated heterocyclic groups such as a furan-2-yl group, furan-3-yl group, thiophen-2-yl group, thiophen-3-yl group, pyrrol-2-yl group, pyrrol-3-yl group, oxazol-2-yl group, oxazol-4-yl group, oxazol-5-yl group, thiazol-2-yl group, thiazol-4-yl group, thiazol-5-yl group, isooxazol-3-yl group, isooxazol-4-yl group, isooxazol-5-yl group, isothiazol-3-yl group, isothiazol-4-yl group, isothiazol-5-yl group, imidazol-2-yl group, imidazol-4-yl group, imidazol-5-yl group, pyrazol-3-yl group, pyrazol-4-yl group, pyrazol-5-yl group, 1,3,4-oxadiazol-2-yl group, 1,3,4-thiadiazol-2-yl group, 1,2,3-triazol-4-yl group, 1,2,4-triazol-3-yl group, 1,2,4-triazol-5-yl group, 5-phenyl-5-trifluoromethyl-isooxazolin-3-yl group, 2-furfurylmethyl group, 3-thienylmethyl group, 1-methyl-3-pyrazolomethyl group, pyridin-2-yl group, pyridin-3-yl group, pyridin-4-yl group, pyridazin-3-yl group, pyridazin-4-yl group, pyrazin-2-yl group, pyrimidin-2-yl group, pyrimidin-4-yl group, pyrimidin-5-yl group, 1,3,5-triazin-2-yl group or 1,2,4-triazin-3-yl group; and saturated heterocyclic groups such as a tetrahydrofuran-2-yl group, tetrahydrofuran-4-yl group, piperidin-3-yl group, pyrrolidin-2-yl group, morpholino group or piperidino group.

Specific examples of the aralkyl group of A include a benzyl group and phenethyl group.

Specific examples of the heteroaralkyl group of A include a 3-thienylmethyl group, 2-pyridylmethyl group, 3-pyridylmethyl group and 2-pyrimidylmethyl group.

Specific examples of substituents on the aromatic hydrocarbon group, heterocyclic group, aralkyl group or heteroaralkyl group of A include the same groups as those listed within a portion of the specific substituent list provided for the alkylene chain of E.

R⁵ represents a hydroxyl group, halogen atom, or unsubstituted or substituted organic group.

Specific examples of the halogen atom of R⁵ include a fluorine atom, chlorine atom, bromine atom and iodine atom.

The organic group of R⁵ represents the all of functional groups including the carbon atom(s), and specific examples include the same groups as those listed within a portion of the specific substituent list provided for the group E, such as a cyano group, alkyl groups, formyl group, amino group, alkenyl groups, alkynyl groups, alkoxy groups, alkenyloxy groups, alkynyloxy groups, aryloxy groups, alkylthiocarbonyl groups, alkylcarbonylamino groups, alkoxycarbonylamino groups, alkoxycarbonyl groups, aromatic hydrocarbon groups, heterocyclic groups, alkylthio groups, alkylsulfonyl groups, alkenylsulfonyl groups, alkynylsulfonyl groups, and arylsulfonyl groups, and provided permitted chemically, these groups may also include a substituent on an atom that constitutes the functional group. Specific examples of these substituents include the same groups as those listed within the specific substituent list provided for the alkylene chain of E.

When the values of e to i, which are described below, are 2 or greater, the plurality of R⁵ groups may be either the same or different. Furthermore, the R⁵ groups may also be linked together to form a ring structure.

The nitrogen atoms within (A-1), (A-3), (A-4) and (A-5) may be bonded directly to the group D, or when not bonded directly to the group D, may be bonded to a hydrogen atom or R⁵ group, provided such bonding is permitted chemically.

A is preferably a group represented by the formulas (A-1) to (A-5) in claims, and specific examples include the groups shown below.

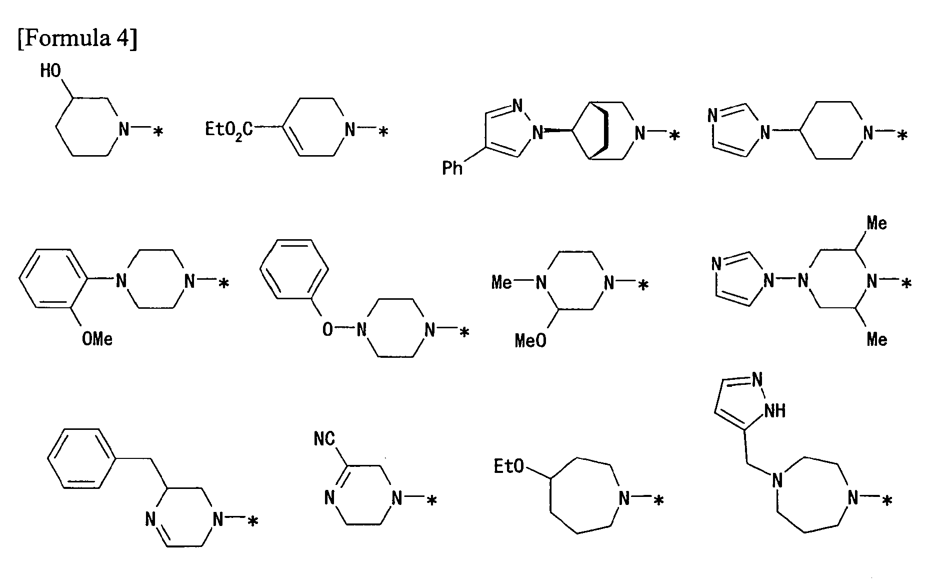

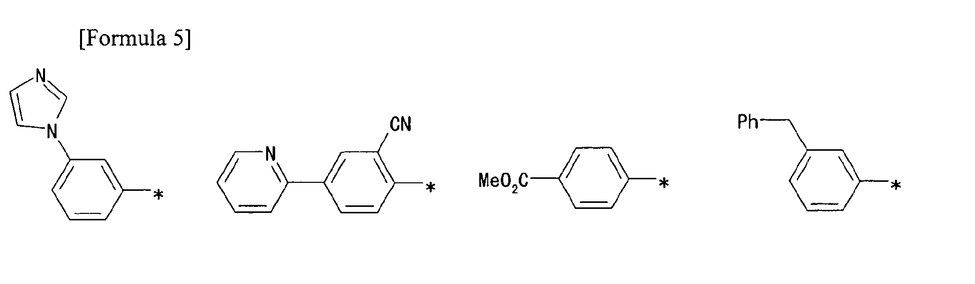

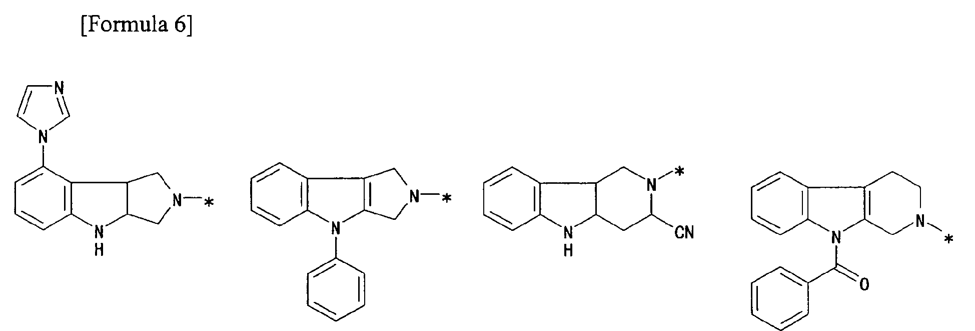

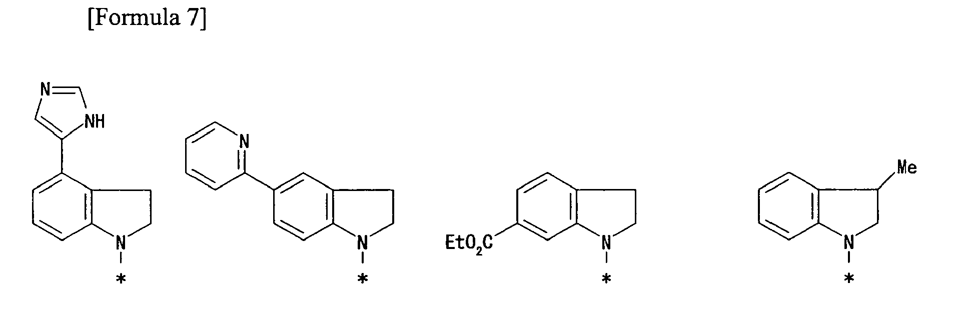

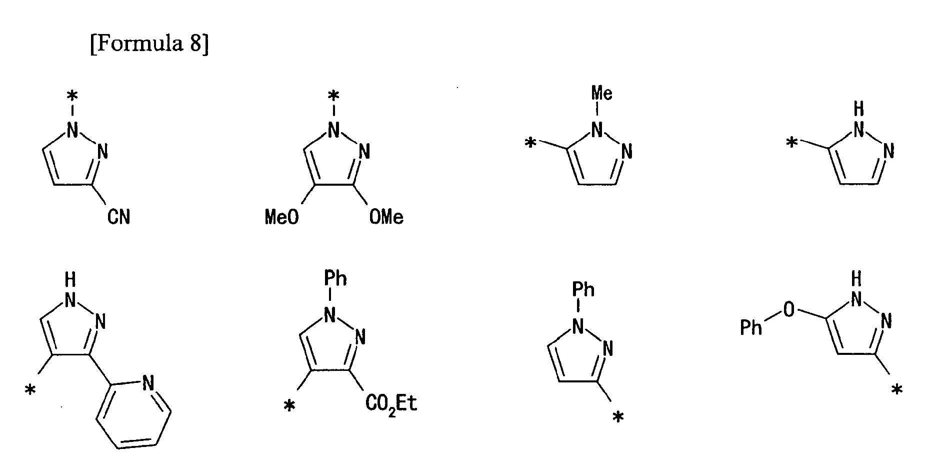

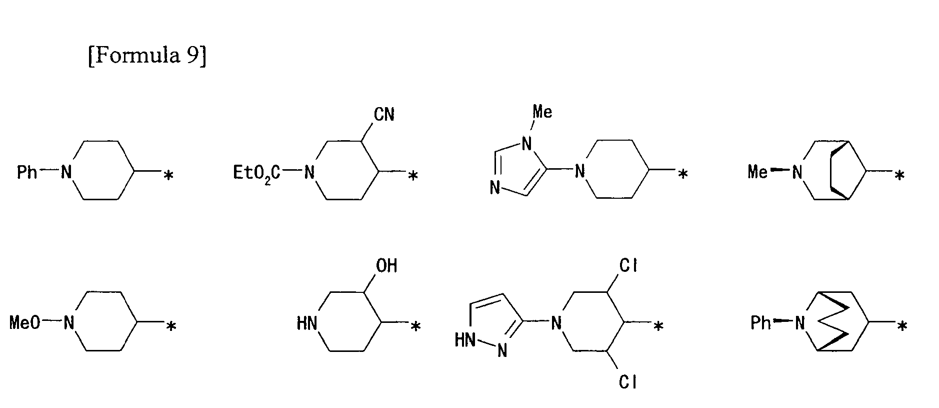

### 2) Production Method

The compound (1) can be produced, for example, using the method shown below. 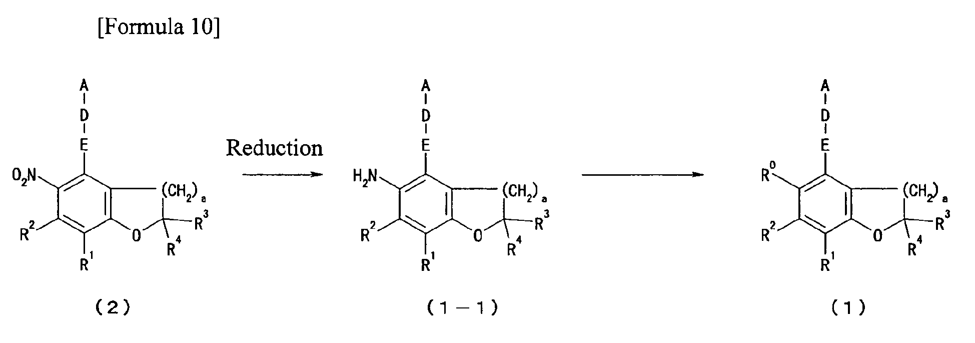 (wherein, R⁰, R¹ to R⁴, E, D, A and a are as defined above)

In other words, the nitro group of the compound represented by the formula (2) (hereafter also referred to as "the compound (2)") is either subjected to hydrogenation in the presence of a hydrogenation catalyst, or reduced using a reducing agent, thereby forming a compound of the present invention represented by the formula (1-1) (hereafter also referred to as "the compound (1-1)").

There are no particular restrictions on the hydrogenation catalyst used in the hydrogenation reaction of the compound (2), and conventional hydrogenation catalysts such as palladium-carbon, palladium hydroxide, platinic oxide and Raney nickel can be used.

This hydrogenation reaction may be conducted within a suitable solvent. There are no particular restrictions on the solvent used, provided it is inert with respect to the reaction. Examples include alcohols such as methanol and ethanol; ethers such as diethyl ether, tetrahydrofuran (hereafter also abbreviated as "THF") and 1,4-dioxane; hydrocarbons such as benzene, toluene, xylene and cyclohexane, amides such as N,N-dimethylformamide (hereafter also abbreviated as "DMF"); organic acids such as formic acid and acetic acid; esters such as ethyl acetate; and mixed solvents containing two or more of the above solvents.

Examples of the method in which a reducing agent is used with the compound (2) include, for example, a method in which reduction is conducted using hydrochloric acid and stannous chloride within an alcohol solvent such as methanol or ethanol; a method in which reduction is conducted using acetic acid and iron within a mixed solvent of water and a ketone such as acetone or methyl ethyl ketone; and a method in which reduction is conducted using ammonium chloride, ammonium acetate, ammonium formate, or acetic acid and zinc, within either an alcohol or a mixed solvent containing an alcohol and water.

In any of the above methods, the reaction temperature is within a range from 0°C to the boiling point of the solvent used.

By converting the amino group of the obtained compound (1-1) to an R⁰ group using a conventional method such as an aniline alkylation, the compound (1) of the present invention can be obtained.

The production intermediate (2) can be produced by typical reactions in the manner described below. 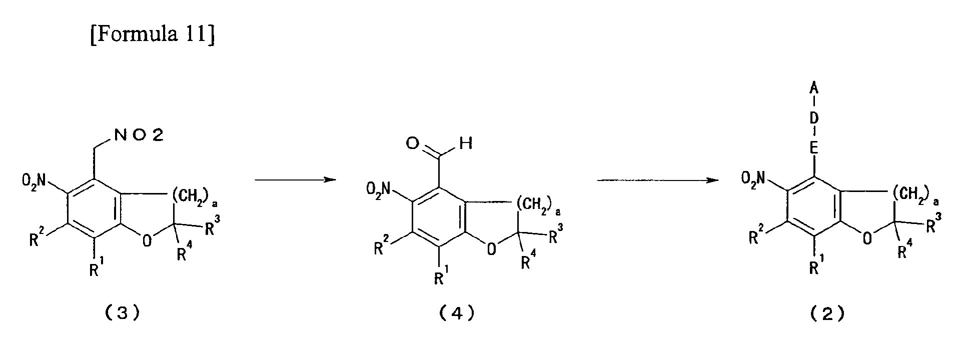 (wherein, R¹ to R⁴, E, D, A and a are as defined above)

In other words, a compound represented by the formula (3) is subjected to a formylation under the action of an oxidizing agent, thereby yielding a compound (4).

More specifically, this reaction can be conducted by adding a base, including an amine such as triethylamine, pyridine or DBU, or an inorganic base such as sodium bicarbonate, sodium carbonate, potassium carbonate or sodium hydroxide, at room temperature to a solution of the compound (3) in an alcohol such as t-butanol, subsequently adding a saturated hydrocarbon such as n-hexane and water to the mixture, also adding an oxidizing agent such as potassium permanganate and boric acid, and then stirring the entire mixture at a temperature within a range from -20°C to +50°C, and preferably from -10°C to +10°C.

In those cases where D is a single bond, the compound (2) can be obtained, for example, by conducting an alkenylation of the compound (4) using a Wittig reaction, and subsequently extending the alkylene chain using a typical reduction reaction.

Furthermore, in those cases where D is an oxygen atom, unsubstituted or substituted nitrogen atom, sulfur atom, sulfinyl group, sulfonyl group or carbonylamino group, the compound (2) can be obtained by reacting the compound (4) with a reducing agent to produce a hydroxyl group, introducing an elimination group if required, and then conducting a typical coupling reaction or the like.

Furthermore, in those cases where D is a carbonyl group or an aminocarbonyl group, the compound (2) can be obtained by converting the compound (4) to a carboxylic acid using a typical oxidizing agent, converting the acid to an acid halide, and then conducting a typical coupling reaction or the like.

The structure of the resulting compound can be identified and confirmed by conducting IR, NMR and MS spectral measurements.

The compound (1) may include optical isomers or tautomers, but all of these isomeric forms are included within the scope of the present invention.

There are no particular restrictions on the salt of the compound (1), provided it is a salt of a compound represented by the formula (1), although pharmacologically permitted salts are preferred. Examples of such salts include salts of inorganic acids such as hydrochloric acid, sulfuric acid, nitric acid and phosphoric acid; and salts of organic acids such as acetic acid, propionic acid, lactic acid, succinic acid, tartaric acid, citric acid, benzoic acid, salicylic acid, nicotinic acid and heptagluconic acid. These salts can be produced easily using conventional chemical synthesis techniques.

### 3) Antioxidant Drug

An antioxidant drug of the present invention includes, as an active ingredient, at least one material selected from the group consisting of compounds represented by the above formula (1) and pharmacologically permitted salts thereof (hereafter referred to as "the compound of the present invention").

The compound of the present invention exhibits excellent antioxidant activity, and by preventing the oxidative degeneration of low density lipoprotein (hereafter also abbreviated as "LDL"), is able to inhibit the generation and progression of arteriosclerosis lesions, and can therefore be used as a drug for the treatment of arteriosclerosis. Furthermore, it is also useful as a therapeutic drug for the various diseases caused by oxidative action, including age-related dementia conditions, heart disease, cancer, diabetes, digestive disorders, burns, eye disease, and renal disease. Moreover, in the case of ischemic organ diseases such as cerebral stroke or myocardial infarction, various active oxygen atoms are generated during reperfusion of the ischemic sites, which can exacerbate tissue damage through cellular membrane breakdown caused by lipid peroxidation reactions, but the compound of the present invention is able remove the various active oxygen and lipid peroxide sites via its antioxidant activity, and can therefore be used as a drug for the treatment of ischemic organ diseases by preventing tissue damage at the ischemic lesions.

Furthermore, the compounds of the present invention also include compounds that have a lipoxygenase inhibiting action or a 20-HETE synthase inhibiting action, and can therefore be used for suppressing the conversion of arachidonic acid to hydroperoxyeicosatetraenoic acid (HPETE) by inhibiting the action of lipoxygenase, or for suppressing the production of 20-HETE by inhibiting the action of 20-HETE synthase.

Furthermore, the compounds of the present invention also include compounds for which the dopamine release-inhibiting action is minimal, reducing the likelihood of side effects in the treatment of Parkinson's disease and the like.

Moreover, the compound of the present invention can also be used in the prevention and treatment of {(a) diseases caused by oxidative lesions of the retina, (b) diabetes, (c) high blood pressure, (d) arteriosclerosis, (e) anemia, (f) leukemia, (g) connective tissue diseases such as systemic lupus erythematosus and scleroderma, (h) vascular disorders, inflammation or degenerative lesions of the retina caused by systemic illnesses including congenital metabolic disorders such as Tay-Sachs disease or Vogt-Spielmeyer disease, (i) retinal vascular disorders such as (retinopathy of prematurity, retinal vein occlusion, retinal artery occlusion and retinal periphlebitis), (j) retinal inflammation or degeneration caused by retinal detachment or external injury, (k) degenerative disorders of the retina accompanying aging, such as age-related macular degeneration, and (1) localized retinal diseases such as congenital retinal degenerative disease}, and is particularly useful as a drug for disorders such as age-related macular degeneration caused by photoinduced oxidative damage.

In an antioxidant drug of the present invention, the therapeutically effective dose of the compound of the present invention varies depending on the individual and the state of the disease being treated. Normally, the therapeutically effective single day dose, per kg of bodyweight, can be set within a range from 0.14 mg to 14.3 mg/day of the compound represented by the formula (1) or the pharmacologically permitted salt thereof, a preferred dose per kg of bodyweight is within a range from 0.7 mg to 10 mg/day, and a particularly preferred dose per kg of bodyweight is from 1.4 mg to 7.2 mg/day. For example, in the case of administration to a 70 kg person, the dosage range for the compound represented by the formula (1) or the pharmacologically permitted salt thereof is from 10 mg to 1.0 g per day, preferably from 50 mg to 700 mg per day, and even more preferably from 100 mg to 500 mg per day, although doses outside this range may be used depending on the state of the disease being treated.

The antioxidant drug of the present invention can be used in the form of a composition which, besides the compound of the present invention that acts as the active ingredient, may also include conventional pharmaceutical carriers or excipients, and other drugs or adjuvants, provided they do not react with the other components. This composition can be formed in accordance with the method of administration to contain from 1 to 99% by weight of the active ingredient, and from 99 to 1% by weight of suitable pharmaceutical carriers or excipients, and preferably contains from 5 to 75% by weight of the active ingredient, with the remainder being suitable pharmaceutical carriers or excipients.

Examples of excipients that can be used in the antioxidant drug of the present invention include all the conventional excipients, such as pharmaceutical mannitol, lactose, starch, gelatinized starch, magnesium stearate, sodium saccharin, talc, cellulose ether derivatives, glucose, gelatin, sucrose, citrates, and propyl gallate. Furthermore, in the case of an antioxidant drug for oral administration, the drug may include diluents such as lactose, sucrose and dicalcium phosphate, disintegrants such as crosscarmellose sodium or derivatives thereof, binders such as magnesium stearate, and lubricants such as starch, gum arabic, polyvinylpyrrolidone, gelatin and cellulose ether derivatives.

The antioxidant drug of the present invention can be administered as a pharmaceutical drug for treating the diseases described above, using all manner of administration systems.

For example, the drug can be administered orally, nasally, parenterally, locally, transdermally or rectally, and the drug may be in the form of a solid, semisolid, lyophilized powder or liquid, which is administered, for example, as a tablet, suppository, pill, soft or hard capsule, medicinal powder, liquid formulation, injectable formulation, suspension, aerosol or sustained-release product. The form is preferably selected to enable accurate administration of the required dose via a simple administration method.

These types of drug products can be produced using normal methods, for example, using the methods disclosed in Remington's Pharmaceutical Sciences 18th edition, published by Mack Publishing Company, Easton, Pennsylvania, 1990.

Injectable formulations may include aseptic aqueous or non-aqueous solvents, suspension agents or emulsifiers. Specific examples of aqueous solvents and suspension diluents include injectable distilled water and physiological saline solution. Specific examples of non-aqueous solvents and suspension diluents include propylene glycol, polyethylene glycol, vegetable oils such as olive oil, alcohols such as ethanol, and polysorbate (a brand name). These compositions may also include other additives such as tonicity agents, preservatives, wetting agents, emulsifiers, dispersants, stabilizers (such as lactose), and solubilizing agents or solubilization assistants. These additives can be produced as a disinfected solid composition by filtration through a bacteria-retaining filter, and then dissolved in aseptic water or an aseptic injection solvent prior to use.

In those cases where the antioxidant drug of the present invention is prepared as a suppository, a carrier is used that dissolves gradually *in vivo,* such as polyoxyethylene glycol or polyethylene glycol (hereafter also abbreviated as "PEG"), or more specifically PEG1000 (96%) or PEG4000 (4%), and from 0.5 to 50% by weight of the compound of the formula (1) or the pharmacologically permitted salt thereof is dispersed within this carrier.

In those cases where the antioxidant drug of the present invention is prepared as a liquid formulation, a saline solution, aqueous dextrose solution, glycerol or ethanol or the like is preferably used as a carrier, and this carrier, together with 0.5 to 50% by weight of the compound of the formula (1) or the pharmacologically permitted salt thereof, and any suitable adjuvant are subjected to a dissolution or dispersion treatment, thereby forming a solution or suspension.

The compound of the present invention is particularly suited to use as a drug for inhibiting photoinduced oxidative lesions of the retina. The administration system, the administered drug form, and the administered dose may use the same administration systems, forms and administration doses as those described above for the antioxidant drug. Furthermore, the same formulation components, carriers and adjuvants and the like as those described above for the antioxidant drug can be included, one or more excipients, disintegrants, binders, or other retinal oxidative lesion inhibitors that do not react with the active ingredient may be added, and other components having different pharmaceutical effects may also be included. Examples of the form in which the drug can be administered include the same drug forms as those described above for the antioxidant drug, as well as eye drops and eye ointments.

In those cases where the drug for inhibiting photoinduced oxidative lesions of the retina according to the present invention is produced as eye drops, one or more of the compounds of the present invention are added to a typical base solvent to form an aqueous solution or suspension, and the pH is then adjusted to a value within a range from 4 to 10, and preferably from 5 to 9. In order to ensure an aseptic product, the eye drops are preferably subjected to a sterilization treatment, and this sterilization treatment may be conducted at any stage within the production process.

The concentration of the compound of the present invention within the eye drops is typically within a range from 0.001 to 3% (w/v), and is preferably from 0.01 to 1% (w/v), and the administered dose can be set to several drops between 1 and 4 times per day, depending on various factors such as the severity of the symptoms and the health of the patient. The above dose is indicative only, and administration may also be conducted at doses exceeding the above range.

The eye drops may also include appropriate quantities of various additives such as buffering agents, tonicity agents, preservatives, pH regulators, thickeners, chelating agents and solubilizing agents, provided they do not react with the compound of the present invention.

Examples of the buffering agents include citrate buffers, tartaric acid buffers, acetate buffers, and amino acids, whereas examples of the tonicity agents include sugars such as sorbitol, glucose and mannitol, polyhydric alcohols such as glycerol, polyethylene glycol, and propylene glycol, and salts such as sodium chloride. Examples of the preservatives include paraoxybenzoate esters such as methyl paraoxybenzoate and ethyl paraoxybenzoate, benzyl alcohol, phenethyl alcohol, and sorbic acid and salts thereof, whereas examples of the pH regulators include phosphoric acid and sodium hydroxide. Examples of the thickeners include hydroxyethylcellulose, hydroxypropylcellulose, methylcellulose, hydroxypropyl methylcellulose, carboxymethylcellulose, and salts thereof, examples of the chelating agents include sodium edetate, sodium citrate and condensed sodium phosphate, and examples of the solubilizing agents include ethanol and polyoxyethylene hardened castor oil.

In those cases where the drug for inhibiting photoinduced oxidative lesions of the retina according to the present invention is produced as an eye ointment, one or more of the compounds of the present invention can be mixed with a typical eye ointment base such as a purified lanolin, white Vaseline, macrogol, plastibase or liquid paraffin, and in order to ensure an aseptic product, the ointment is preferably subjected to a sterilization treatment.

The concentration of the compound of the present invention within the eye ointment is typically within a range from 0.001 to 3% (w/v), and is preferably from 0.01 to 1% (w/v), and the administered dose may involve application between 1 and 4 times per day, depending on various factors such as the severity of the symptoms and the health of the patient. The above dose is indicative only, and administration may also be conducted at doses exceeding the above range.

Because the drug for inhibiting photoinduced oxidative lesions of the retina according to the present invention exhibits a superior antioxidant action, it is effective, for example, for the prevention and treatment of degenerative disorders of the retina that accompany aging, such as age-related macular degeneration.

### EXAMPLES

A more detailed description of the present invention based on a series of examples is provided below, although the technical scope of the present invention is in no way limited by the following examples.

### (Example 1) Production of 2,2,6,7-tetramethyl-4-phenylethyl-5-aminodihydrobenzofuran

Step 1: Production of 2,2,6,7-tetramethyl-5-nitrodihydrobenzofuran-4-aldehyde

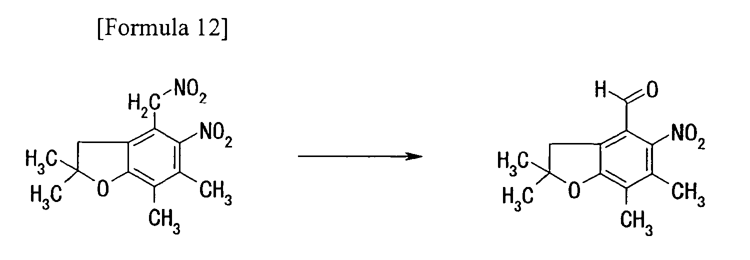

0.4 g of 60% sodium hydride was added to 50 ml of t-butanol, and the mixture was stirred for 10 minutes at room temperature. To this mixture was added a solution of 0.80 g of 2,2,6,7-tetramethyl-4-nitromethyl-5-nitrodihydrobenzofuran dissolved in 80 ml of t-butanol, and the resulting mixture was stirred for 20 minutes at room temperature. To the resulting solution was added a solution containing 0.80 g of potassium permanganate and 0.63 g of boric acid dissolved in 1,200 ml of n-hexane and 50 ml of water, and the entire solution was stirred for 10 minutes at room temperature. Sodium thiosulfate was then added to the reaction solution, and stirring was continued until the reaction liquid became colorless. The thus obtained reaction liquid was extracted with n-hexane, washed with a saturated saline solution, and dried over anhydrous magnesium sulfate, and the solvent was then removed by evaporation under reduced pressure, yielding 0.71 g of a crude product of the target compound.

Step 2: Production of 2,2,6,7-tetramethyl-4-styryl-5-nitrodihydrobenzofuran

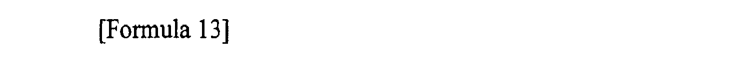

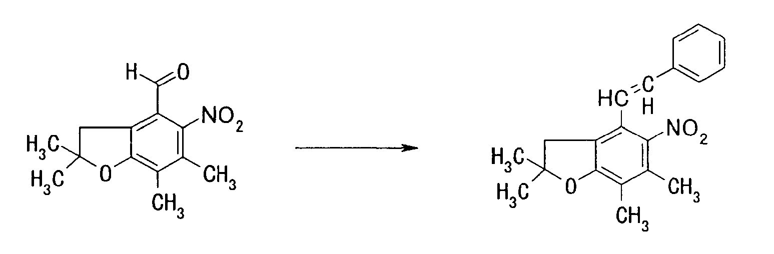

2.1 g of diethyl benzylphosphonate was added to 38 ml of THF, 30 mg of 60% sodium hydride was added, and the entire mixture was stirred for 30 minutes at room temperature. 1.9 g of the 2,2,6,7-tetramethyl-5-nitrodihydrobenzofuran-4-aldehyde was then added, and the entire mixture was stirred overnight at room temperature. The reaction liquid was then poured into ice water, and extracted with ethyl acetate. The organic layer was dried over anhydrous magnesium sulfate, and the solvent was then removed by evaporation under reduced pressure. The residue was purified by silica gel column chromatography (developing solvent: benzene/n-hexane = 1/1 (volume ratio)), yielding 1.4 g of the target compound.

Step 3: Production of 2,2,6,7-tetramethyl-4-phenylethyl-5-aminodihydrobenzofuran

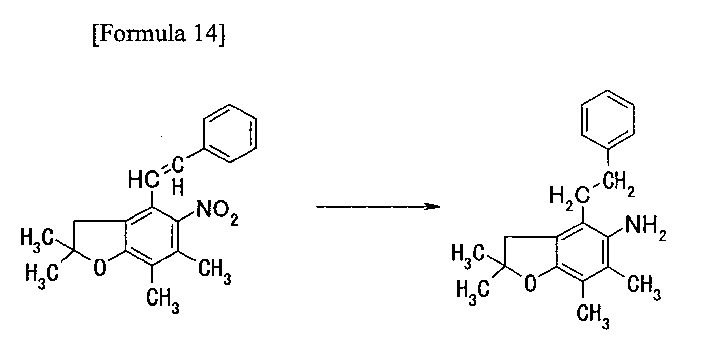

0.70 g of the 2,2,6,7-tetramethyl-4-styryl-5-nitrodihydrobenzofuran was dissolved in 20 ml of methanol, 0.3 g of 10% palladium-carbon was added, and the entire mixture was stirred for 3 hours at 70°C under a hydrogen pressure of 1 MPa. The reaction liquid was then subjected to celite filtration, and the filtrate was concentrated under reduced pressure, yielding 0.75 g of the target compound.
Refractive index: n_{D}^{20.5} 1.5467
¹H-NMR (CDCl₃, δ ppm):
1.3 (s, 6H), 2.1 (s, 6H), 2.7 to 2.9 (m, 4H), 3.2 (br, 2H), 7.1 to 7.3 (m, 5H)
(Example 2) Production of 4-(5-amino-2,2,6,7-tetramethyldihydrobenzofuran-4-ylmethyl)-1-[(4-imidazol-1-yl)phenylmethyl]piperazine

Step 1: Production of 4-(5-nitro-2,2,6,7-tetramethyldihydrobenzofuran-4-ylmethyl)-1-[(4-imidazol-1-yl)phenylmethyl]piperazine

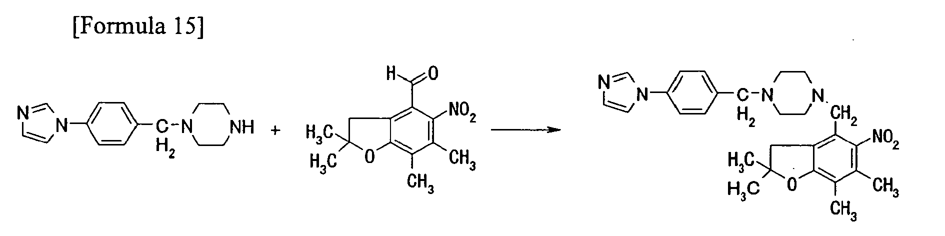

0.54 g of 1-[4-imidazol-1-yl)phenylmethyl]piperazine and 0.50 g of 2,2,6,7-tetramethyl-5-nitrodihydrobenzofuran-4-aldehyde were dissolved in 17 ml of methylene chloride. To the resulting solution was added 0.25 ml of acetic acid, and the mixture was stirred for one hour at room temperature. 0.85 g of sodium triacetoxyborohydride was added to the reaction liquid, and the entire mixture was stirred for one day at room temperature. The reaction liquid was then poured into water, neutralized with an aqueous solution of sodium hydroxide, and then extracted with chloroform. The organic layer was washed with a saturated saline solution, and dried over anhydrous magnesium sulfate, and the solvent was then concentrated under reduced pressure. The residue was purified by silica gel column chromatography (developing solvent: chloroform/ethyl acetate = 9/1 → = chloroform/methanol = 20/1 (volume ratio)), yielding 0.51 g of the target compound.

Step 2: Production of 4-(5-amino-2,2,6,7-tetramethyldihydrobenzofuran-4-ylmethyl)-1-[(4-imidazol-1-yl)phenylmethyl]piperazine

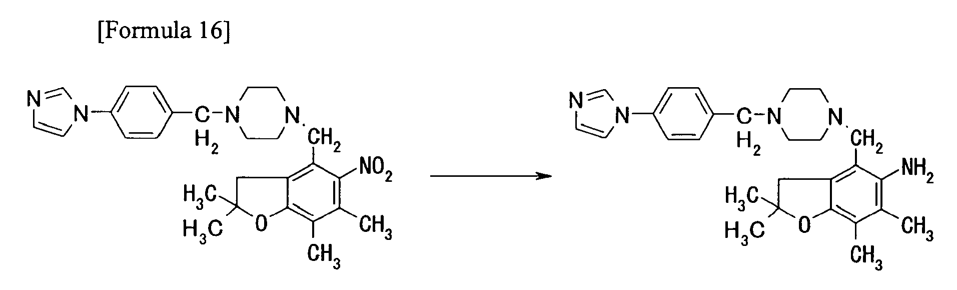

0.51 g of the 4-(5-nitro-2,2,6,7-tetramethyldihydrobenzofuran-4-ylmethyl)-1-[(4-imidazol-1-yl)phenylmethyl]piperazine and 2.3 g of ammonium chloride were added to a mixed solvent containing 45 ml of ethanol and 12 ml of water, 3.5 g of zinc powder was then added, and the entire mixture was stirred for 30 minutes at room temperature. Following completion of the reaction, the reaction liquid was filtered, and the solvent was removed from the filtrate by evaporation under reduced pressure. Chloroform was added to the thus obtained residue, the resulting solution was washed sequentially with a saturated aqueous sodium bicarbonate solution and a saturated saline solution, and following drying over anhydrous magnesium sulfate, the solution was concentrated under reduced pressure. The residue was purified by silica gel column chromatography (developing solvent: chloroform/methanol = 20/1 (volume ratio)), yielding 0.40 g of the target compound.
Melting point: 187 to 190°C
(Example 3) Production of 5-(4-(5-amino-2,2,6,7-tetramethyldihydrobenzofuran-4-ylmethyl)aminophenyl)pyrazole

Step 1: Production of 5-(4-(5-nitro-2,2,6,7-tetramethyldihydrobenzofuran-4-ylmethyl)aminophenyl)pyrazole

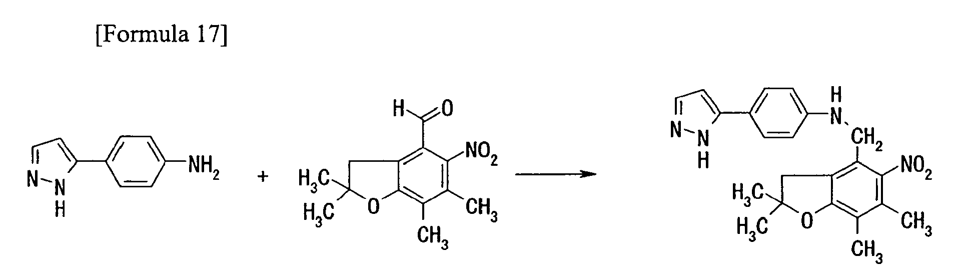

4.80 g of 5-(4-aminophenyl)pyrazole and 5.00 g of 2,2,6,7-tetramethyl-5-nitrodihydrobenzofuran-4-aldehyde were dissolved in 180 ml of methylene chloride. To the resulting solution was added 21.4 ml of acetic acid, and the entire mixture was refluxed for 15 hours. Following cooling of the reaction liquid to room temperature, tetrahydrofuran was added to dissolve the insoluble matter, the resulting solution was washed sequentially with a saturated aqueous sodium bicarbonate solution and a saturated saline solution, and following drying over anhydrous magnesium sulfate, the solvent was removed by evaporation under reduced pressure. The residue was dissolved in a mixed solvent containing 80 ml of methanol and 50 ml of tetrahydrofuran, 3.04 g of sodium tetrahydroborate was added, and the entire mixture was stirred for 4 hours at room temperature. A saturated saline solution was added to the resulting reaction liquid, and the mixture was extracted 3 times with chloroform. The extract was washed sequentially with 1N hydrochloric acid, a 1N aqueous solution of sodium hydroxide, and a saturated saline solution, and following drying over anhydrous magnesium sulfate, the solvent was removed by evaporation under reduced pressure. The residue was purified by silica gel column chromatography (developing solvent: chloroform/methanol = 20/1 (volume ratio)), yielding 5.92 g of the target compound.

Step 2: Production of 5-(4-(5-amino-2,2,6,7-tetramethyldihydrobenzofuran-4-ylmethyl)aminophenyl)pyrazole

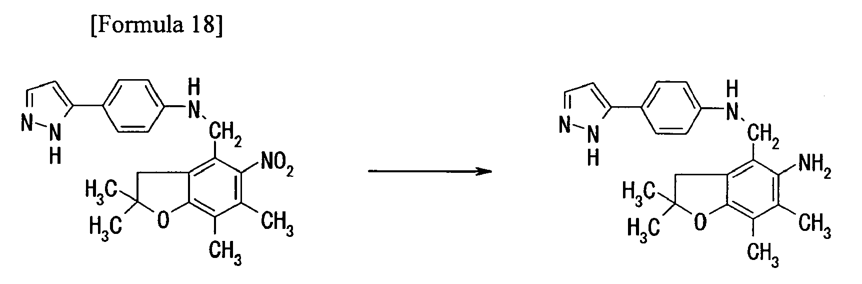

0.66 g of the 5-(4-(5-nitro-2,2,6,7-tetramethyldihydrobenzofuran-4-ylmethyl)aminophenyl)pyrazole was dissolved in 29 ml of acetic acid, and with the solution cooled in an ice bath, 2.9 g of zinc powder was added gradually. Following completion of the addition, the entire mixture was stirred for 15 minutes at room temperature. Following completion of the reaction, the reaction liquid was filtered, and following washing of the solid with methanol, the solvent was removed from the filtrate by evaporation under reduced pressure. Chloroform was added to the thus obtained residue, the resulting solution was washed sequentially with a 1N aqueous solution of sodium hydroxide and a saturated saline solution, and following drying over anhydrous magnesium sulfate, the solution was concentrated under reduced pressure. The residue was purified by silica gel column chromatography (developing solvent: chloroform/methanol = 20/1 (volume ratio)), yielding 0.44 g of the target compound.
Melting point: 166 to 169°C
(Example 4) Production of 5-(4-(5-amino-2,2,6,7-tetramethyldihydrobenzofuran-4-ylmethyl)-N-acetylaminophenyl)pyrazole

Step 1: Production of 5-(4-(5-nitro-2,2,6,7-tetramethyldihydrobenzofuran-4-ylmethyl)aminophenyl)-1-(tetrahydropyran-2-yl)pyrazole

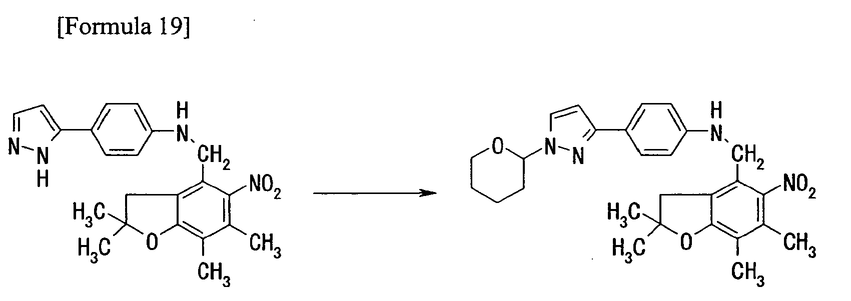

2.87 g of 5-(4-(5-nitro-2,2,6,7-tetramethyldihydrobenzofuran-4-ylmethyl)aminophenyl)pyrazole and 0.06 g of p-toluenesulfonic acid hydrate were dissolved in 20 ml of tetrahydrofuran, and the entire mixture was heated to 50°C. A solution containing 0.92 g of 3,4-dihydro-2H-pyran dissolved in 20 ml of tetrahydrofuran was then added dropwise to the reaction solution over a period of 30 minutes, and the resulting mixture was then stirred for 19.5 hours at 55°C. During this period, additional 2 ml samples of 3,4-dihydro-2H-pyran were added after 5 hours and after 7.5 hours respectively. Following cooling, the reaction liquid was washed with 19 ml of 3N ammonia water, and the organic layer was then washed with water until the pH reached 7. The organic layer was dried over anhydrous magnesium sulfate, and then concentrated under reduced pressure. The residue was purified by silica gel column chromatography (developing solvent: hexane/ethyl acetate = 2/1 (volume ratio)), yielding 3.10 g of the target compound.

Step 2: Production of 5-(4-(5-nitro-2,2,6,7-tetramethyldihydrobenzofuran-4-ylmethyl)-N-acetylaminophenyl)-1-(tetrahydropyran-2-yl)pyrazole

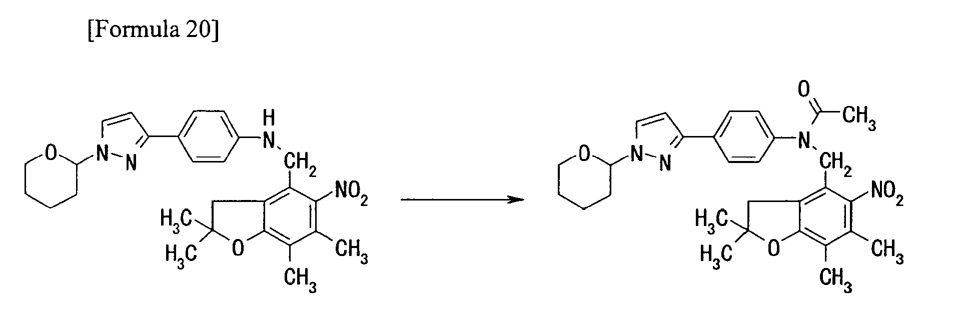

0.90 g of the 5-(4-(5-nitro-2,2,6,7-tetramethyldihydrobenzofuran-4-ylmethyl)aminophenyl)-1-(tetrahydropyran-2-yl)pyrazole was dissolved in 5 ml of methylene chloride. To the resulting solution were added 0.23 g of triethylamine and 0.18 g of acetyl chloride, and the entire mixture was stirred for 1.5 hours at room temperature. The reaction liquid was then washed sequentially with a saturated aqueous sodium bicarbonate solution and a saturated saline solution, and following drying over anhydrous magnesium sulfate, the solvent was removed by evaporation under reduced pressure. The residue was purified by silica gel column chromatography (developing solvent: chloroform/ethyl acetate = 9/1 (volume ratio)), yielding 0.72 g of the target compound.

Step 3: Production of 5-(4-(5-amino-2,2,6,7-tetramethyldihydrobenzofuran-4-ylmethyl)-N-acetylaminophenyl)pyrazole

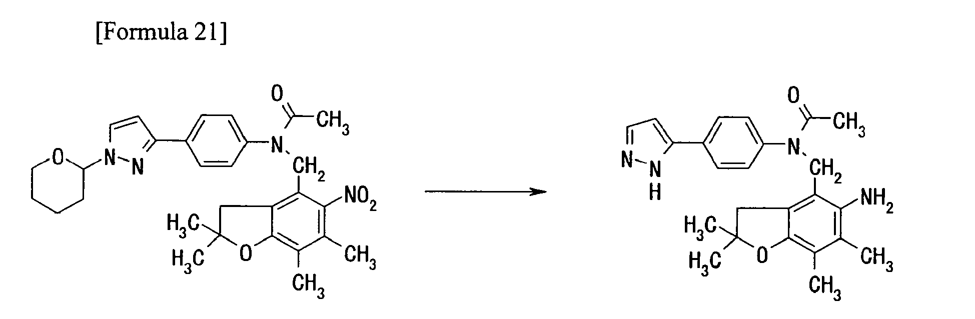

0.72 g of the 5-(4-(5-nitro-2,2,6,7-tetramethyldihydrobenzofuran-4-ylmethyl)-N-acetylaminophenyl)-1-(tetrahydropyran-2-yl)pyrazole was dissolved in 22 ml of methanol. To the resulting solution was added 11 ml of 6N hydrochloric acid, and the entire mixture was then stirred for 2.5 hours at room temperature. The reaction liquid was neutralized with an aqueous solution of sodium hydroxide, and then extracted twice with chloroform. The organic layer was washed with a saturated saline solution, and following drying over anhydrous magnesium sulfate, the solvent was removed by evaporation under reduced pressure. The residue was dissolved in 24 ml of acetic acid, and with the solution cooled in an ice bath, 0.91 g of zinc powder was added gradually. Following completion of the addition, the entire mixture was stirred for 15 minutes at room temperature. Following completion of the reaction, the reaction liquid was filtered, and following washing of the solid with chloroform, the solvent was removed from the filtrate by evaporation under reduced pressure. Chloroform was added to the thus obtained residue, the resulting solution was washed sequentially with a 1N aqueous solution of sodium hydroxide and a saturated saline solution, and following drying over anhydrous magnesium sulfate, the solution was concentrated under reduced pressure. The residue was purified by silica gel column chromatography (developing solvent: chloroform/methanol = 20/1 (volume ratio)), yielding 0.15 g of the target compound.
Melting point: 196 to 199°C
(Example 5) Production of 5-(4-(5-amino-2,2,6,7-tetramethyldihydrobenzofuran-4-ylmethoxy)phenyl)pyrazole

Step 1: Production of 2,2,6,7-tetramethyl-5-nitrodihydrobenzofuran-2-ylmethyl methanesulfonate

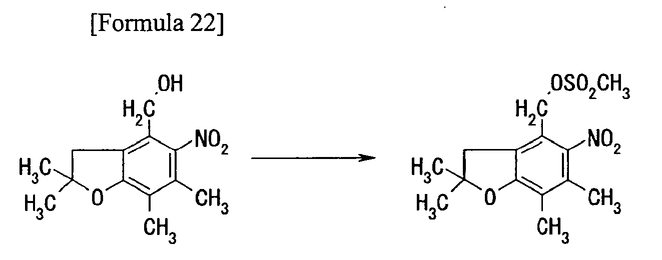

2.00 g of 2,2,6,7-tetramethyl-4-hydroxymethyl-5-nitrodihydrobenzofuran was dissolved in 33 ml of toluene. To the resulting solution were added 1.61 g of triethylamine and a solution containing 0.96 g of methanesulfonyl chloride dissolved in 5 ml of toluene, and the entire mixture was then stirred for 4 hours at room temperature. The reaction liquid was then filtered, the solid was washed with toluene, and the solvent was removed by evaporation under reduced pressure, yielding 3.06 g of the target compound.

Step 2: Production of 4'-(5-nitro-2,2,6,7-tetramethyldihydrobenzofuran-4-ylmethoxy)acetophenone

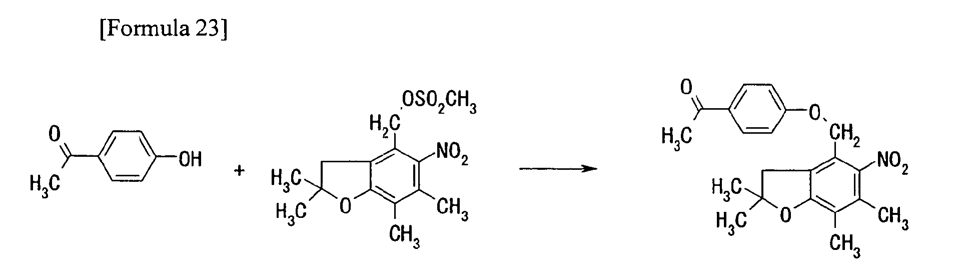

0.50 g of 4'-hydroxyacetophenone was dissolved in 5 ml of N,N-dimethylformamide. The resulting solution was cooled to 0°C, 0.16 g of 60% sodium hydride was added, and the entire mixture was stirred for 30 minutes at 0°C. To the reaction mixture was then added a solution containing 1.00 g of the 2,2,6,7-tetramethyl-5-nitrodihydrobenzofuran-2-ylmethyl methanesulfonate dissolved in 5 ml of N,N-dimethylformamide, and the entire mixture was stirred for 30 minutes at 0°C, and then for a further 7 hours at room temperature. The reaction liquid was then poured into ice water, and extracted three times with ether. The organic layer was washed three times with water and dried over anhydrous magnesium sulfate, and the solvent was then removed by evaporation under reduced pressure, yielding 1.00 g of the target compound.

Step 3: Production of 5-(4-(5-nitro-2,2,6,7-tetramethyldihydrobenzofuran-4-ylmethoxy)phenyl)pyrazole

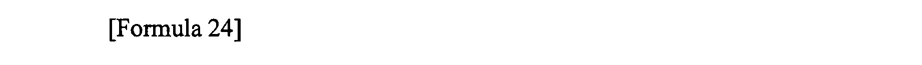

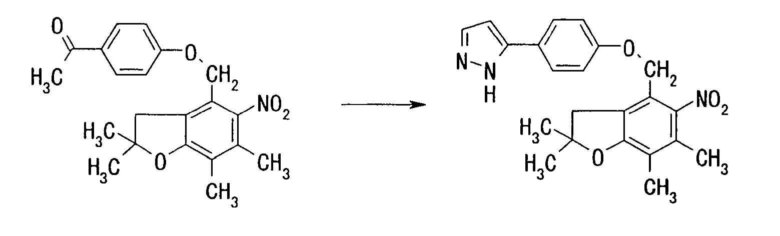

1.00 g of the 4'-(5-nitro-2,2,6,7-tetramethyldihydrobenzofuran-4-ylmethoxy)acetophenone was dissolved in 6 ml of N,N-dimethylformamide. To the resulting solution was added 6 ml of N,N-dimethylformamide dimethyl acetal, and the entire mixture was heated under reflux for 14 hours. Following completion of the reaction, the solvent was removed by evaporation under reduced pressure. The residue was dissolved in 12 ml of ethanol, 0.20 g of p-toluenesulfonic acid hydrate and 1.6 ml of hydrazine hydrate were added, an the entire mixture was heated under reflux for 2.5 hours. Following completion of the reaction, the solvent was removed by evaporation under reduced pressure. Chloroform was added to the thus obtained residue, the resulting solution was washed sequentially with a 1N aqueous solution of sodium hydroxide and a saturated saline solution, and following drying over anhydrous magnesium sulfate, the solvent was removed by evaporation under reduced pressure. The residue was purified by silica gel column chromatography (developing solvent: chloroform/ethyl acetate = 4/1 (volume ratio)), yielding 0.95 g of the target compound.

Step 4: Production of 5-(4-(5-amino-2,2,6,7-tetramethyldihydrobenzofuran-4-ylmethoxy)phenyl)pyrazole

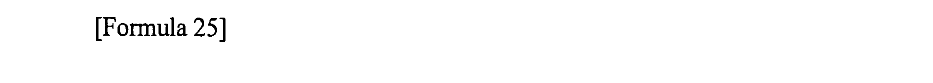

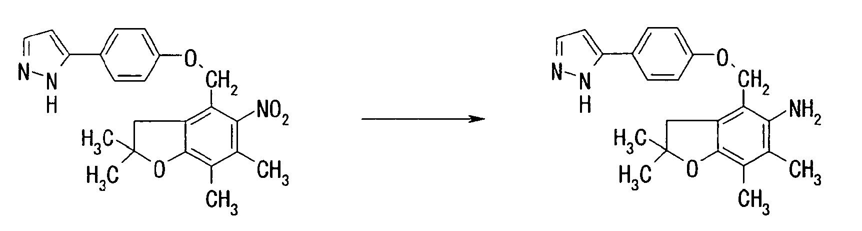

0.95 g of the 5-(4-(5-nitro-2,2,6,7-tetramethyldihydrobenzofuran-4-ylmethoxy)phenyl)pyrazole was dissolved in 41 ml of acetic acid, and with the solution cooled in an ice bath, 1.58 g of zinc powder was added gradually. Following completion of the addition, the entire mixture was stirred for 30 minutes at room temperature. Following completion of the reaction, the reaction liquid was filtered, and following washing of the solid with chloroform, the solvent was removed from the filtrate by evaporation under reduced pressure. Chloroform was added to the thus obtained residue, the resulting solution was washed sequentially with a 1N aqueous solution of sodium hydroxide and a saturated saline solution, and following drying over anhydrous magnesium sulfate, the solution was concentrated under reduced pressure. The residue was purified by silica gel column chromatography (developing solvent: chloroform/methanol = 20/1 (volume ratio)), and the solvent was removed by evaporation under reduced pressure. 1N hydrochloric acid was added to the residue, the mixture was washed 3 times with chloroform, 1N sodium hydroxide was added to the water phase, and the product was extracted with chloroform and washed with a saturated saline solution. The resulting organic phase was dried over anhydrous magnesium sulfate and then concentrated under reduced pressure, yielding 0.30 g of the target compound.
Melting point: 131 to 135°C
(Example 6) Production of 1-(1-(5-amino-2,2,6,7-tetramethyldihydrobenzofuran-4-ylmethyl)piperidin-4-yl)-3-phenylpyrazole

Step 1: Production of 1-tert-butoxycarbonylpiperidin-4-yl methanesulfonate

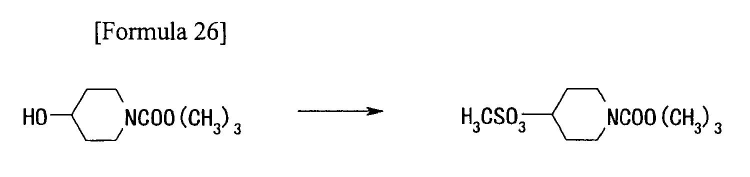

5.42 g of 1-tert-butoxycarbonyl-4-hydroxypiperidine was dissolved in 54 ml of toluene. To the resulting solution were added 5.44 g of triethylamine and 3.24 g of methanesulfonyl chloride, and the entire mixture was stirred for one hour at room temperature. The reaction liquid was then filtered, and following washing of the solid with toluene, the solvent was removed from the filtrate by evaporation under reduced pressure, yielding 7.54 g of the target compound.

Step 2: Production of 1-(1-tert-butoxycarbonylpiperidin-4-yl)-3-phenylpyrazole

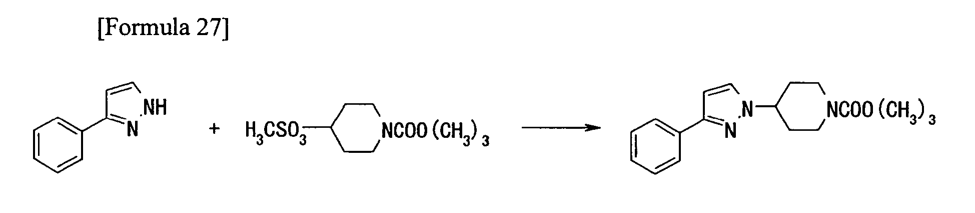

1.57 g of 3-phenylpyrazole was dissolved in 11 ml of N,N-dimethylformamide. To the resulting solution was added 0.50 g of 60% sodium hydride, and the entire mixture was stirred for 1.5 hours at room temperature. To this mixture was added a solution containing 2.90 g of the 1-tert-butoxycarbonylpiperidin-4-yl methanesulfonate dissolved in 2 ml of N,N-dimethylformamide, and the entire mixture was stirred for two hours at 50°C, one hour at 70°C, and then 3.5 hours at 100°C. The reaction liquid was then poured into water, and extracted twice with ether. The organic layer was washed three times with water and dried over anhydrous magnesium sulfate, and the solvent was then removed by evaporation under reduced pressure. The residue was purified by silica gel column chromatography (developing solvent: hexane/ethyl acetate = 4/1 (volume ratio)), yielding 1.54 g of the target compound.

Step 3: Production of 1-(piperidin-4-yl)-3-phenylpyrazole

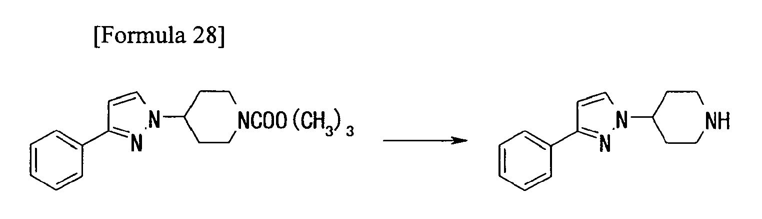

To 1.54 g of the 1-(1-tert-butoxycarbonylpiperidin-4-yl)-3-phenylpyrazole was added 15 ml of 35% hydrochloric acid, and the entire mixture was then stirred for 9 hours at room temperature. Following completion of the reaction, an aqueous solution of sodium hydroxide was added to neutralize the reaction liquid, and the mixture was extracted three times with a mixed solvent of chloroform and methanol. The organic layer was washed with a saturated saline solution and dried over anhydrous magnesium sulfate, and the solvent was then removed by evaporation under reduced pressure, yielding 1.09 g of the target compound.

Step 4: Production of 1-(1-(5-nitro-2,2,6,7-tetramethyldihydrobenzofuran-4-ylmethyl)piperidin-4-yl)-3-phenylpyrazole

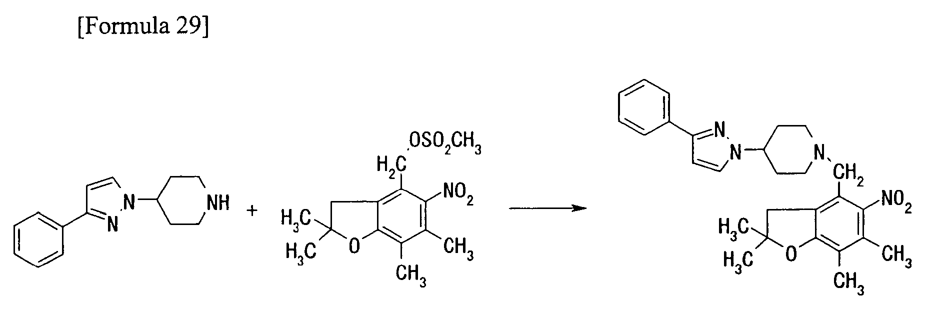

0.92 g of 2,2,6,7-tetramethyl-5-nitrodihydrobenzofuran-2-ylmethyl methanesulfonate and 0.58 g of the 1-(piperidin-4-yl)-3-phenylpyrazole were dissolved in 9 ml of acetonitrile. To the resulting solution was added 0.30 g of sodium carbonate, and the entire mixture was then refluxed under heat for 13 hours. Following reaction, the solvent was removed by evaporation under reduced pressure, chloroform was added, the resulting solution was washed sequentially with water and a saturated saline solution and then dried over anhydrous magnesium sulfate, and the solvent was then removed by evaporation under reduced pressure. The residue was purified by silica gel column chromatography (developing solvent: hexane/ethyl acetate = 4/1 (volume ratio)), yielding 1.00 g of the target compound.

Step 5: Production of 1-(1-(5-amino-2,2,6,7-tetramethyldihydrobenzofuran-4-ylmethyl)piperidin-4-yl)-3-phenylpyrazole

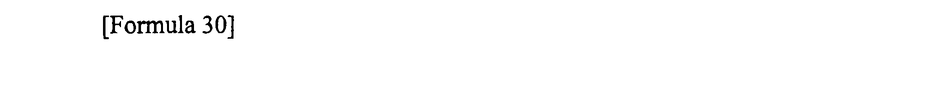

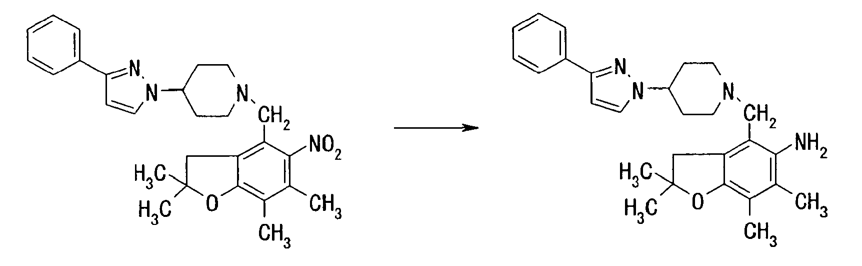

1.00 g of the 1-(1-(5-nitro-2,2,6,7-tetramethyldihydrobenzofuran-4-ylmethyl)piperidin-4-yl)-3-phenylpyrazole was dissolved in 37 ml of acetic acid, and with the solution cooled in an ice bath, 1.42 g of zinc powder was added gradually. Following completion of the addition, the entire mixture was stirred for 30 minutes at room temperature. Following completion of the reaction, the reaction liquid was filtered, and following washing of the solid with chloroform, the solvent was removed from the filtrate by evaporation under reduced pressure. Chloroform was added to the thus obtained residue, the resulting solution was washed sequentially with a 1N aqueous solution of sodium hydroxide and a saturated saline solution, and following drying over anhydrous magnesium sulfate, the solution was concentrated under reduced pressure. The residue was purified by silica gel column chromatography (developing solvent: chloroform/ethyl acetate = 3/2 (volume ratio)), yielding 0.65 g of the target compound.
Melting point: 134 to 136°C

Examples of the compound (1) produced in the manner described above are shown below in Table 1.
In the table, for those compounds for which the column titled "Physical Constants" includes the expression "&NMR", NMR data are provided below the table.
Furthermore, the expression "n_{D}^{20.8} 1.5852" means that the refractive index at a temperature of 20.8°C is 1.5852 (this also applies to similar expressions).
The abbreviations used in the table represent the groups listed below.
Me: methyl group, Et: ethyl group, iPr: isopropyl group, nBu: normal butyl group, tBu: tertiary butyl group, Ph: phenyl group, Ac: acetyl group, and Bn: benzyl group.

[Table 1]

**Table 1**

| | | | | | | | | |
|---|---|---|---|---|---|---|---|---|
| 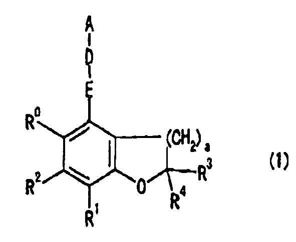 | | | | | | | | |

| Compound No. | a | R⁰ | R¹ | R² | R³ | R⁴ | A-D-E | Physical Constants: []m.p°C n_{D} Refractive Index |
|---|---|---|---|---|---|---|---|---|
| 1 | 1 | NH₂ | Me | Me | Me | Me | 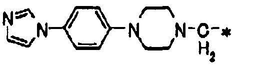 | [172-174] |
| 2 | 1 | NH₂ | Me | H | H | Me | 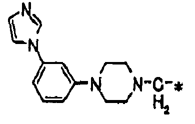 | |
| 3 | 2 | NH₂ | Me | Me | Me | Me | 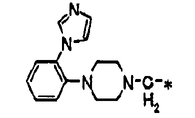 | |
| 4 | 1 | NAc₂ | Me | Me | Me | Me | 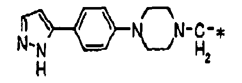 | |
| 5 | 1 | NH₂ | Me | Me | Me | Me | 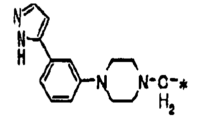 | |
| 6 | 1 | NHC(=O)Ph | Me | Me | Me | Me | 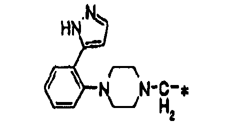 | |
| 7 | 1 | NH₂ | Me | Me | Me | Me | 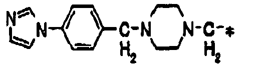 | [187-190] |
| 8 | 1 | NHCO₂Me | Me | Me | Me | Me | 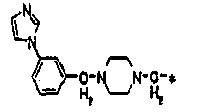 | |
| 9 | 1 | NMe₂ | Me | Me | Me | Me | 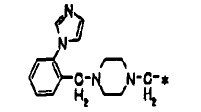 | |
| 10 | 1 | NH₂ | Me | Me | Me | Me | 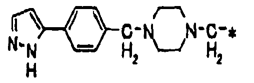 | [145-147] |
| 11 | 1 | NH₂ | Me | Me | Me | Me | 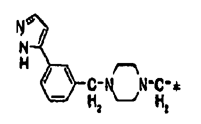 | |
| 12 | 2 | NH₂ | Me | Me | Me | Me | 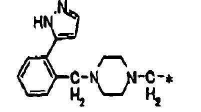 | |
| 13 | 2 | NH₂ | Me | Me | Me | Me | 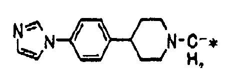 | |
| 14 | 1 | NH₂ | Et | Me | H | H | 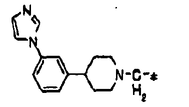 | |
| 15 | 1 | NH₂ | Me | Me | Me | Me | 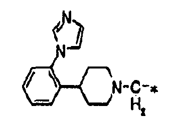 | |
| 16 | 1 | NH₂ | Me | Me | Me | Me | 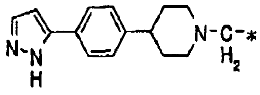 | Amorphous &NMR |
| 17 | 1 | NHBn | Me | Me | Me | Me | 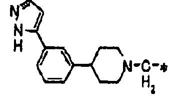 | |
| 18 | 1 | NH₂ | Me | Me | Me | Me | 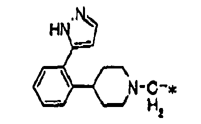 | |
| 19 | 1 | NH₂ | H | H | H | H | 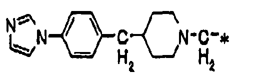 | |
| 20 | 1 | NH₂ | Me | Me | Me | Me | 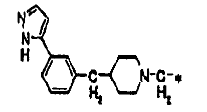 | |
| 21 | 1 | NH₂ | Me | Me | Me | Me | 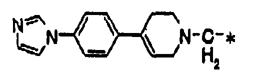 | |
| 22 | 1 | NH₂ | Me | Me | Me | Me | 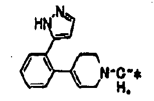 | |
| 23 | 1 | NH₂ | Me | Me | Me | Me | 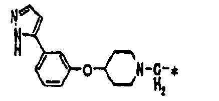 | |
| 24 | 1 | NH₂ | Me | Me | Me | Me | 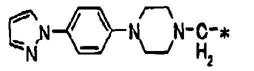 | [163-164] |
| 25 | 1 | NH₂ | Me | Me | Me | Me | 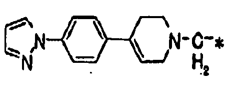 | Amorphous &NMR |
| 26 | 1 | NH₂ | Me | Me | Me | Me | 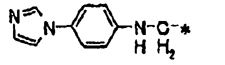 | [193-195] |
| 27 | 1 | NH₂ | Me | Me | Me | Me | 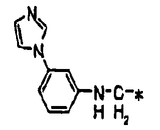 | [125-127] |
| 28 | 1 | NH₂ | Me | Me | Me | Me | 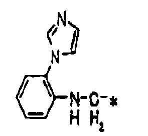 | [142-144] |
| 29 | 1 | NH₂ | Me | Me | Me | Me | 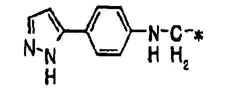 | [166-169] |
| 30 | 1 | NH₂ | Me | Me | Me | Me | 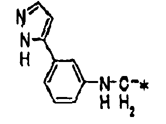 | [182-163] |
| 31 | 1 | NH₂ | Me | Me | Me | Me | 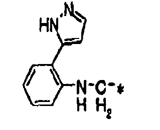 | [150-152] |
| 32 | 1 | NH₂ | Me | Me | Me | Me | 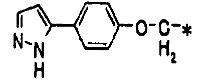 | [131-135] |
| 33 | 1 | NH₂ | Me | Me | Me | Me | 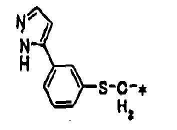 | |
| 34 | 1 | NH₂ | Me | Me | nBu | nBu | 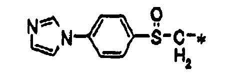 | |
| 35 | 2 | NH₂ | Me | Me | Me | Me | 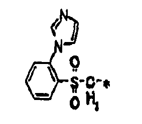 | |
| 36 | 2 | NH₂ | Me | Me | Me | Me | 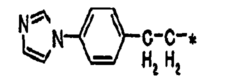 | |
| 37 | 1 | NH₂ | H | Me | Et | Et | 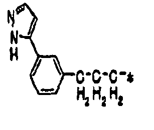 | |
| 38 | 1 | NtBu₂ | Me | Me | Me | Me | 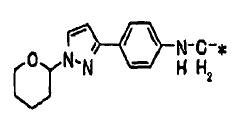 | |
| 39 | 1 | NH₂ | Me | Me | Me | Me | 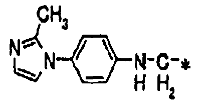 | [193-196] |
| 40 | 1 | NH₂ | Me | Me | Me | Me | 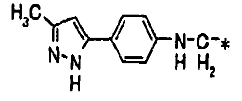 | [167-170] |
| 41 | 1 | NH₂ | Me | Me | Me | Me | 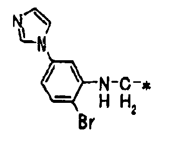 | [153-155] |
| 42 | 1 | NH₂ | Me | Me | Me | Me | 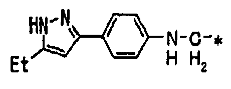 | [195-197] |
| 43 | 1 | NH₂ | Me | Me | Me | Me | 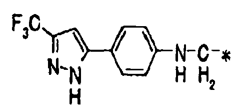 | [201-203] |
| 44 | 1 | NH₂ | Et | Et | H | H | 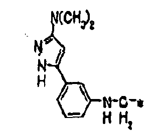 | |
| 45 | 1 | NH₂ | Me | Me | Me | Me | 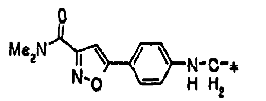 | |
| 46 | 1 | NH₂ | Me | Me | Me | Me | 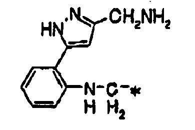 | |
| 47 | 1 | NH₂ | Me | Me | Me | Me | 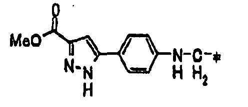 | [178-180] |
| 48 | 1 | NH₂ | Me | Me | Me | Me | 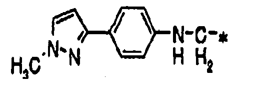 | [183-186] |
| 49 | 1 | NH₂ | Me | Me | Me | Me | 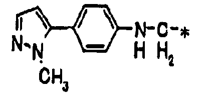 | [181-184] |
| 50 | 1 | NH₂ | H | Et | Me | Me | 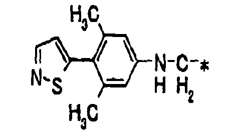 | |
| 51 | 1 | NHAo | Me | Me | Me | Me | 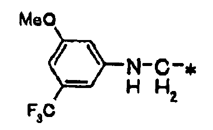 | |
| 52 | 1 | NH₂ | Me | Me | Me | Me | 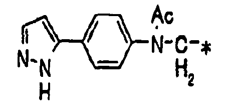 | [196-199] |
| 53 | 1 | NH₂ | Me | Me | H | Me | 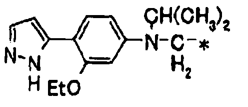 | |
| 54 | 1 | NH₂ | Me | Me | Me | Me | 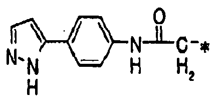 | |
| 55 | 1 | NH₂ | Me | Me | Et | Et | 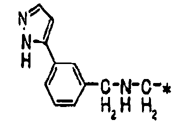 | |
| 56 | 2 | NH₂ | Me | Me | Me | Me | 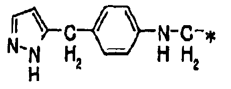 | |
| 57 | 1 | NH₂ | Me | Me | Me | Me | 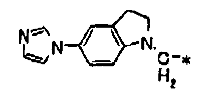 | [148-152] |
| 58 | 1 | NH₂ | Me | Me | Me | Me | 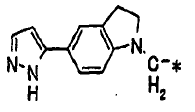 | [119-121] |
| 59 | 1 | NH₂ NH₂ | Me | Me | Me | Me | 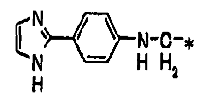 | [204-206] |
| 60 | 1 | NH₂ | Me | Me | Me | Me | 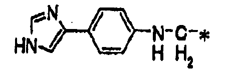 | [225-227] |
| 61 | 1 | NH₂ | Me | Me | Me | Me | 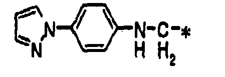 | [180-182] |
| 62 | 1 | NH₂ | Me | Me | Me | Me | 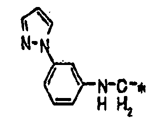 | [145-147] |
| 63 | 1 | NH₂ | Me | Me | Me | Me | 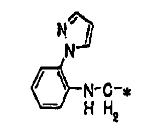 | [126-128] |
| 64 | 1 | NH₂ | Me | Me | Me | Me | 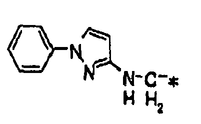 | [190-193] |
| 65 | 1 | NH₂ | Me | Me | Me | Me | 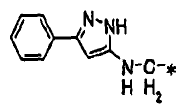 | [113-116] |
| 66 | 1 | NH₂ | Me | Me | Me | Me | 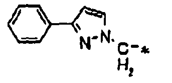 | [156-158] |
| 67 | 1 | NH₂ | Me | Me | Me | Me | 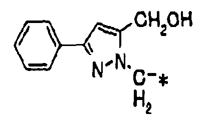 | [284-289] |
| 68 | 1 | NH₂ | Me | Me | Me | Me | 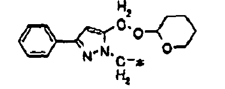 | [175-176] |
| 69 | 1 | NH₂ | Me | Me | Me | Me | 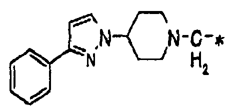 | [134-136] |
| 70 | 1 | NH₂ | Me | Me | Me | Me | 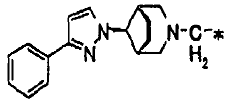 | [194-197] |
| 71 | 2 | NH₂ | H | H | H | H | 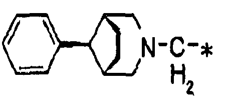 | |
| 72 | 1 | NH₂ | Me | Me | Me | Me | 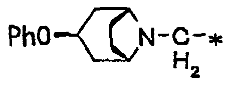 | |
| 73 | 1 | NH₂ | Me | Me | Me | Me | 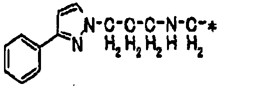 | nD^{20.8}1.5852 |
| 74 | 1 | NH₂ | Me | Me | Me | Me | 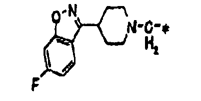 | [174-176] |
| 75 | 1 | NH₂ | Me | Me | Me | Me | 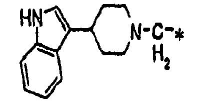 | [160-162] |
| 76 | 1 | NH₂ | Me | Me | Me | Me | 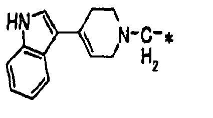 | [150-152] |
| 77 | 1 | NH₂ | Me | Me | Me | Me | 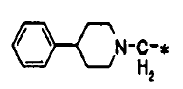 | [163-165] |
| 78 | 1 | NHPh | iPr | Me | Me | Me | 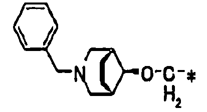 | |
| 79 | 1 | NH₂ | Me | Me | Me | Me | 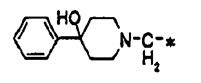 | |
| 80 | 1 | NH₂ | Me | Me | Me | Me | 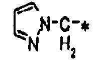 | [141-143] |
| 81 | 1 | NH₂ | Me | Me | Me | Me | 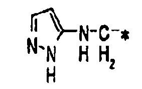 | [178-182] |
| 82 | 1 | NH₂ | Me | Me | Me | Me | 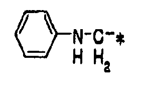 | [124-125] |
| 83 | 1 | NH₂ | Me | Me | Me | Me | 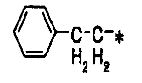 | nD^{20.5}1.5467 |
| 84 | 1 | NH₂ | Me | Me | Me | Me | 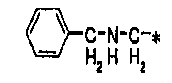 | [80-82] |
| 85 | 1 | NH₂ | Me | Me | Me | Me | 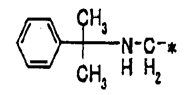 | [62-64] |
| 86 | 1 | NH₂ | Me | Me | Me | Me | 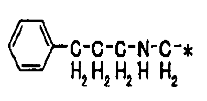 | nD^{22.2}1.5665 |
| 87 | 1 | NH₂ | Me | Me | Me | Me | 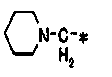 | nD^{21.3}1.5450 |
| 88 | 1 | NH₂ | Me | Me | Me | Me | 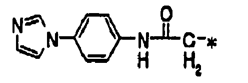 | |
| 89 | 1 | NH₂ | Me | Me | tBu | H | 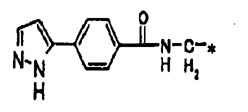 | |
| 90 | 2 | NH₂ | H | H | Me | Me | 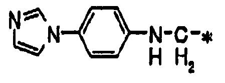 | |
| 91 | 1 | NH₂ | Me | Me | Me | Me | 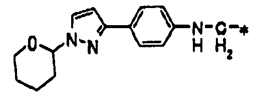 | [165-168] |

¹H-NMR (CDCl₃, δ ppm)
Compound 16: 1.5 (s, 6H), 1.6-1.9 (m, 4H), 2.05 (m, 2H), 2.1 (s, 3H), 2.2 (s, 3H), 2.5 (m, 1H), 2.9 (s, 2H), 3.0 (d, 2H), 3.5 (s, 2H), 6.5 (d, 1H), 7.3 (d, 2H), 7.6 (d, 1H), 7.7 (d, 2H) Compound 25: 1.5 (m, 8H), 2.1 (s, 3H), 2.2 (s, 3H), 2.5 (br, 2H), 2.7 (m, 2H), 3.0 (s, 2H), 3.2 (m, 2H), 3.5 (s, 2H), 6.1 (m, 1H), 6.5 (m, 1H), 7.5 (d, 2H), 7.65 (d, 2H), 7.75 (d, 1H), 7.9 (d, 1H), 8.8 (s, 1H)
Compound 73: 1.4 (s, 6H), 2.0-2.1 (m, 2H), 2.09 (s, 3H), 2.13 (s, 3H), 2.6 (t, 2H), 2.9 (s, 2H), 3.1 (br, 2H), 3.7 (s, 2H), 4.2 (t, 2H), 6.5 (d, 1H), 7.25 (d, 1H), 7.22-7.30 (m, 1H), 7.35-7.41 (m, 2H), 7.8 (d, 2H)
Compound 83: 1.3 (s, 6H), 2.1 (s, 6H), 2.7-2.9 (m, 4H), 3.2 (br, 2H), 7.1-7.3 (m, 5H) Compound 86: 1.4 (s, 6H), 1.76-1.86 (m, 2H), 2.06 (s, 3H), 2.12 (s, 3H), 2.63-2.68 (m, 4H), 2.9 (s, 2H), 3.7 (s, 2H), 7.13-7.21 (m, 3H), 7.28 (t, 2H)
Compound 87: 1.4 (s, 6H), 1.49-1.56 (m, 6H), 2.05 (s, 3H), 2.12 (s, 3H), 2.4 (br, 4H), 2.9 (s, 2H), 3.3 (s, 2H), 4.4 (br, 2H)

### {Preparation of Drug Formulation}

A drug formulation containing the compound of the present invention was prepared using the method described below.
Drug Formulation Example 1: Preparation of an Oral Drug (a tablet with an active ingredient of 10 mg)
Compound of the present invention: 10 mg
Lactose: 81.4 mg
Corn starch: 20 mg
Hydroxypropylcellulose: 4 mg
Carboxymethylcellulose calcium: 4 mg
Magnesium stearate: 0.6 mg
Total: 120 mg

In order to achieve the composition listed above, 50 g of the compound of the present invention, 407 g of lactose, and 100 g of corn starch were mixed uniformly using a flow coater (manufactured by Okawara Corporation). 200g of a 10% aqueous solution of hydroxypropylcellulose was then sprayed onto the mixture, generating granules. Following drying, the granules were passed through a 20-mesh sieve, 20 g of carboxymethylcellulose calcium and 3 g of magnesium stearate were added, and a tableting machine (manufactured by Hata Iron Works Co., Ltd.) with a pestle of 7 mm x 8.4R was used to form tablets with a weight of 120 mg per tablet.

### (Test for Evaluating in vitro Inhibition of Lipid Peroxidation)

Evaluation of the *in vitro* inhibition of lipid peroxidation of the compound of the present invention was conducted in accordance with the method disclosed by Malvy et al. (Malvy, C., et al., Biochemical and Biophysical Research Communications, 1980, vol. 95, pp. 734 to 737).

As the organ, retinas that had been separated from pig eyeballs and subsequently stored at -80°C were used. At the time of use, a 5-fold excess of a phosphate-buffered physiological saline solution (pH 7.4) was added, and the mixture was homogenized using a microhomogenizer (PHYSCOTRON, NITI-ON).
To this pig retina homogenate were added 0.15M KCl, the compound of the present invention, 500 pM cysteine and 5pM FeSO₄, and the mixture was then incubated for 30 minutes at 37°C.
The malondialdehyde generated upon decomposition of the lipid peroxide was measured using the thiobarbituric acid method. Based on the measured values, the 50% inhibition concentration (hereafter also referred to as "IC₅₀") of the compound of the present invention was determined.
The results are shown in the table below.
It is evident that the compound of the present invention exhibited an *in vitro* inhibition of lipid peroxidation.

**[Table 2]**

| Compound Number | *in vitro* inhibition of lipid peroxidation (50% inhibition concentration IC₅₀: µM) |
|---|---|
| 1 | 0.36 |
| 7 | 0.37 |
| 16 | 0.34 |
| 26 | 0.27 |
| 27 | 0.29 |
| 28 | 0.30 |
| 29 | 0.36 |
| 30 | 0.27 |
| 31 | 0.30 |
| 32 | 0.28 |
| 39 | 0.31 |
| 40 | 0.33 |
| 43 | 0.30 |
| 47 | 0.31 |
| 48 | 0.27 |
| 49 | 0.26 |
| 57 | 0.33 |
| 59 | 0.30 |
| 60 | 0.32 |
| 61 | 0.28 |
| 62 | 0.33 |
| 63 | 0.30 |
| 64 | 0.30 |
| 65 | 0.31 |
| 66 | 0.35 |
| 68 | 0.30 |
| 69 | 0.31 |
| 70 | 0.25 |
| 73 | 0.33 |
| 74 | 0.29 |
| 83 | 0.26 |
| 84 | 0.25 |
| 85 | 0.33 |
| 86 | 0.31 |
| R-1 | 0.30 |

### (Test for Evaluating Tissue Distribution)

The tissue distribution properties of the compound of the present invention were evaluated by measuring the *ex vivo* inhibition of lipid peroxidation.
The compound of the present invention was dissolved in DMSO (final concentration: 20%), and then dissolved or suspended in a 0.1N hydrochloric acid physiological saline solution or a 1% polyethylene hardened castor oil (NIKOL HCO-60, manufactured by Nikko Chemicals Co., Ltd.)
This solution was administered orally, at a dose of 30 mg/kg, to either a group of three SD male rats (Japan SLC, 6 weeks old) that had been feed-deprived for 24 hours, or a group of three SD male rats (Japan SLC, 6 weeks old) that had not been feed-deprived. One hour after administration and following anesthetization, the brains and eyeballs were removed. Using the method disclosed above in the section relating to the "test for evaluating *in vitro* inhibition of lipid peroxidation ", the brains, and the retinas separated from the eyeballs were homogenized, and the quantity of lipid peroxide within each tissue homogenate was then measured.

The inhibition rate of the compound of the present invention within each of the tissues was determined from the quantities of lipid peroxide generated in a control group (20% DMSO-0.1N hydrochloric acid physiological saline solution / 1% polyethylene hardened castor oil) and in the group administered with the compound of the present invention. The results are shown in the table below. From the following table it is evident that the compound of the present invention exhibits a high degree of tissue distribution into the retinas and the brain.

**[Table 3]**

| Compound Number | *ex vivo* inhibition of lipid peroxidation 50% inhibition quantity (ID₅₀: mg/kg) | ex vivo inhibition of lipid peroxidation inhibition rate (%) |
|---|---|---|
| | Retina | Brain |
| 29 | 60 (with feeding) | - |
| 40 | 24 (with feeding) | - |
| 83 | 67 (with feeding) | 96 (fasting) |
| R-1 | 72 (with feeding) | - |

### (Test for Evaluating Oral Absorption)

The absorption of the compound of the present invention upon oral administration was evaluated in rats.
The compound of the present invention was dissolved in DMSO (final concentration: 20%), and then dissolved or suspended in a 0.1N hydrochloric acid physiological saline solution or a 1% polyethylene hardened castor oil (NIKOL HCO-60, manufactured by Nikko Chemicals Co., Ltd.).
This solution was administered orally, at a dose of 30 mg/kg, to a group of three SD male rats (Japan SLC, 6 weeks old). One hour after administration and following anesthetization, blood was collected from the abdominal vena cava in a plastic blood collection tube containing a serum separating agent. The blood sample was left to stand for approximately one hour, and following clotting, the serum was obtained by conducting a centrifugal separation at 3,000 rpm for a period of 10 minutes. To a 0.3 ml sample of the serum was added 0.9 ml of acetonitrile, and following mixing, the mixture was centrifuged for 10 minutes at 12,000 rpm, and the resulting supernatant phase was filtered (chromatodisc 13P). The quantity of the compound of the present invention that existed within this filtered solution was measured by high-performance liquid chromatography (HPLC), under the conditions shown below. The filtered solution for HPLC analysis was stored at -20°C until the point of measurement.

The filtered solution for HPLC described in the previous paragraph was measured using the following representative conditions.
HPLC: HPLC system from JASCO Corporation
Column: Inertsil ODS-3 (manufactured by GL Sciences Inc.)
Mobile phase: acetonitrile / 10 mM phosphate buffer pH 6.8 = 70/30
Flow rate: 1 ml/minute
Measurement wavelength: 254 nm

A previously prepared calibration curve for the compound of the present invention was used to calculate the blood concentration level one hour after oral administration to the rats. The results are shown in the table below. From the following table it is evident that the compound of the present invention exhibited a high level of oral absorption.

**[Table 4]**

| Compound Number | Test for evaluating oral absorption: serum concentration (µM) |
|---|---|
| 29 | 2.4 |
| R-1 | Below detection limit |

In each of the tests, a compound (R-1) of the formula shown below, disclosed in International Patent Publication No. 2004/092153 pamphlet, was used as a control.

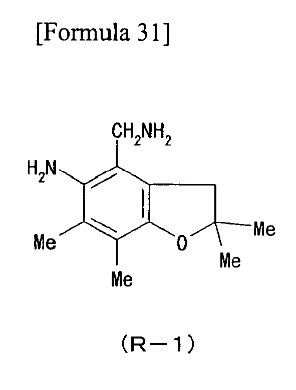

### INDUSTRIAL APPLICABILITY

A compound of the present invention represented by the above formula (1) or a salt thereof exhibits effective antioxidant activity in the treatment of ischemic organ disorders such as arteriosclerosis, myocardial infarction and brain infarction, and in the treatment of diseases caused by oxidative cell damage such as renal disease. Accordingly, the compound or salt can effectively inhibit retinal lesions caused by oxidation due to the effects of light or the like, enabling the preparation of an excellent antioxidant drug containing the ortho-substituted aniline derivative of the present invention. Furthermore, the antioxidant drug of the present invention can also be used as an oxidative lesion inhibitor with minimal side effects, a lipoxygenase inhibitor, a 20-HETE synthase inhibitor, a drug for treating renal disease, cerebrovascular disease or cardiovascular disease, and a drug for treating brain infarction and the like.

## Claims

1. A compound represented by a formula (1), or a salt thereof: 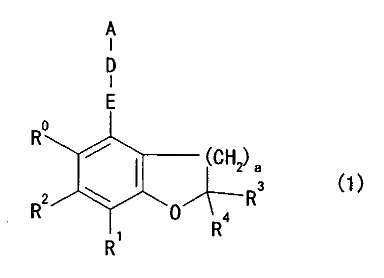 {where in said formula (1):
a represents either 1 or 2,
R⁰ represents an unsubstituted or substituted amino group,
R¹ to R⁴ each represent, independently, a hydrogen atom or an alkyl group,
E represents an unsubstituted or substituted alkylene chain,
D represents a single bond, oxygen atom, unsubstituted or substituted nitrogen atom, sulfur atom, sulfinyl group, sulfonyl group, carbonyl group, carbonylamino group or aminocarbonyl group, and
A represents an unsubstituted or substituted aromatic hydrocarbon group, unsubstituted or substituted heterocyclic group, unsubstituted or substituted aralkyl group, or unsubstituted or substituted heteroaralkyl group}.

2. A compound or salt thereof according to claim 1, wherein said A is a group represented by one of formulas (A-1) to (A-5) shown below: 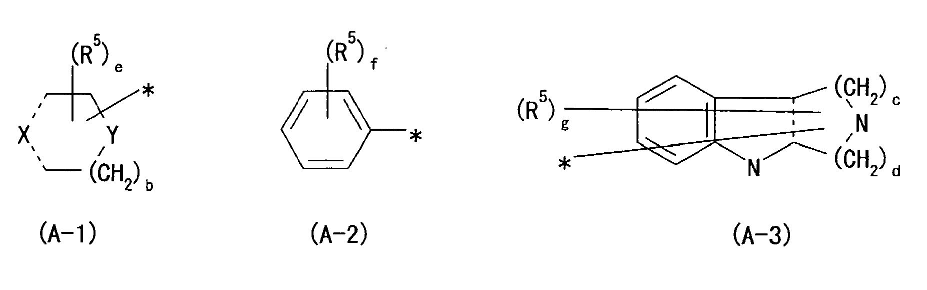 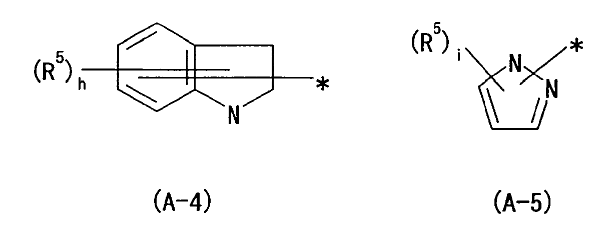 {where in said formulas (A-1) to (A-5):
R⁵ represents a hydroxyl group, halogen atom, or unsubstituted or substituted organic group, and when e to i are 2 or greater, a plurality of R⁵ groups may be either identical or different, or may be linked together to form a ring structure,
b, c and d each represent, independently, either 1 or 2,
e represents an integer from 0 to 9,
f represents an integer from 0 to 5,
g represents an integer from 0 to 7,
h represents an integer from 0 to 8,
i represents an integer from 0 to 3,
X and Y each represent, independently, a carbon atom, oxygen atom, or nitrogen atom, provided that X and Y are not both carbon atoms,
a dotted line in said formula (A-1) represents either a single bond or a double bond when X is a carbon atom, and both dotted lines can not represent double bonds simultaneously, and represents a single bond when X is an oxygen atom or nitrogen atom,
a dotted line in said formula (A-3) represents either a single bond or a double bond, and * represents a bonding position}.

3. An antioxidant drug comprising, as an active ingredient, at least one material selected from the group consisting of compounds represented by a formula (1) and salts thereof: 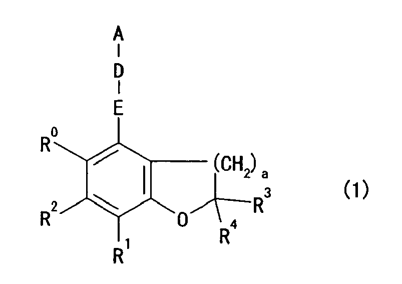 {where in said formula (1):
a represents either 1 or 2,
R⁰ represents an unsubstituted or substituted amino group,
R¹ to R⁴ each represent, independently, a hydrogen atom or an alkyl group,
E represents an unsubstituted or substituted alkylene chain,
D represents a single bond, oxygen atom, unsubstituted or substituted nitrogen atom, sulfur atom, sulfinyl group, sulfonyl group, carbonyl group, carbonylamino group or aminocarbonyl group, and
A represents an unsubstituted or substituted aromatic hydrocarbon group, unsubstituted or substituted heterocyclic group, unsubstituted or substituted aralkyl group, or unsubstituted or substituted heteroaralkyl group}.

4. An antioxidant drug according to claim 3, which is a renal disease therapeutic drug, cerebrovascular disease therapeutic drug, cardiovascular disease therapeutic drug, brain infarction therapeutic drug, or retinal oxidative lesion inhibitor.

5. An antioxidant drug according to claim 4, wherein said retinal oxidative lesion inhibitor is a therapeutic drug for age-related macular degeneration, or a therapeutic drug for diabetic retinopathy.
